# EUROPÄISCHE PATENTSCHRIFT

(11) **EP 3 585 764 B1**
(45) Veröffentlichungstag und Bekanntmachung des Hinweises auf die Patenterteilung: **27.01.2021**
(21) Anmeldenummer: 18704024.1
(22) Anmeldetag: 13.02.2018
(51) Int. Cl.: C07C 67/08, C07C 51/09, C07C 51/12, C07C 51/353, C07C 57/00, C07C 57/03, C07C 57/26, C07C 69/52, C07D 307/92, C12P 7/40, C12P 7/62

(54) **VERFAHREN ZUR HERSTELLUNG VON UNGESÄTTIGTEN CARBONSÄUREN DURCH CARBONYLIERUNG VON ALLYLALKOHOLEN UND DEREN ACYLIERUNGSPRODUKTEN**
PROCESS FOR THE PREPARATION OF UNSATURATED CARBOXYLIC ACIDS BY CARBONYLATION OF ALLYL ALCOHOLS AND THEIR ACYLATION PRODUCTS
PROCÉDÉ DE PRÉPARATION D'ACIDES CARBOXYLIQUES INSATURÉS PAR CARBONYLATION D'ALCOOLS ALLYLIQUES ET LEURS PRODUITS D'ACYLATION

(30) Priorität: 24.02.2017 EP 17157950
(43) Veröffentlichungstag der Anmeldung: 01.01.2020
(73) Patentinhaber: BASF SE, 67056 Ludwigshafen am Rhein (DE)
(72) Erfinder: SCHELWIES, Mathias, 67056 Ludwigshafen (DE); PACIELLO, Rocco, 67056 Ludwigshafen (DE); PELZER, Ralf, 68623 Lampertheim (DE); SIEGEL, Wolfgang, 67056 Ludwigshafen (DE)
(86) Internationale Anmeldenummer: PCT/EP2018/053535
(87) Internationale Veröffentlichungsnummer: WO 2018/153727

(56) Entgegenhaltungen:
- EP-A2- 0 428 979
- DE-A1- 2 217 534
- DE-C1- 4 301 555
- US-A- 6 015 923

## Beschreibung

### HINTERGRUND DER ERFINDUNG

Die vorliegende Erfindung betrifft ein Verfahren zur Carbonylierung von Allylalkoholen bei niedriger Temperatur, niedrigem Druck und/oder niedriger Katalysatorbeladung. Die vorliegende Erfindung betrifft weiterhin die Herstellung von Folgeprodukten dieser Carbonylierungsprodukte und speziell von (-)-Ambrox.

### STAND DER TECHNIK

Die Carbonylierungsprodukte von Allylakoholen sind wertvolle Zwischenprodukte zur Herstellung einer Vielzahl kommerzieller Produkte. Durch Carbonylierung der Allylalkohole Nerolidol und Farnesol kann man beispielsweise Carbonsäuren erhalten, die nach Reduktion zu den entsprechenden Alkoholen zu wertvollen Aromachemikalien zyklisiert werden können. So kann beispielsweise die (3E,7E)-Homofarnesylsäure aus der Carbonylierung von E-Nerolidol einer Reduktion unter Erhalt von (3E,7E)-Homofarnesol 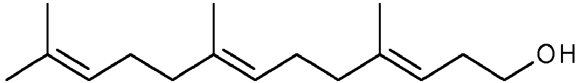 und dieses weiter einer Zyklisierung unter Erhalt von (-)-Ambrox 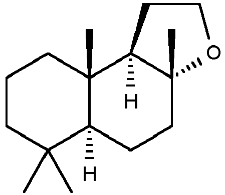 unterzogen werden.

Verfahren zur Carbonylierung von Allylalkoholen zu ihren C1-verlängerten linearen Carbonsäuren oder Carbonsäureestern in Gegenwart von homogenen und auch heterogenen Katalysatoren sind aus der Literatur bekannt. Diese Verfahren sind jedoch aus verschiedenen Gründen wirtschaftlich nachteilig. So ist beispielsweise bekannt, dass die direkte Carbonylierung von Allylalkoholen harsche Bedingungen bezüglich Temperatur und Druck und hohe Katalysatorbeladungen benötigt, in den meisten Fällen sind zudem auch beim Einsatz hoher Drücken oder Temperaturen oder Katalysatorbeladungen hohe Umsätze überhaupt nur unter Zusatz von halogenidhaltigen Additiven erreichbar. Es besteht daher ein Bedarf an einem Verfahren, das die ökonomisch vorteilhafte Herstellung der Carbonylierungsprodukte von Allylalkoholen ermöglicht.

Bertleff, W., Roeper, M. and Sava, X. 2007. beschreiben in Ullmann's Encyclopedia of Industrial Chemsitry, Bd. 7, Seiten 74-98, 2012, Wiley-VCH, industriell relevante Methoden zur Carbonylierung.

Tsuji et al. beschreiben in J. Am. Chem. Soc. 86, 1964, 4350-4353 die katalytische Carbonylierung von Allylverbindungen unter Einsatz von Palladium(II)-chlorid. Konkret werden Allylchlorid, Allylalkohol und analoge Verbindungen mit CO in organischen Lösungsmittel umgesetzt, wobei mit Ethanol die entsprechenden Carbonsäureester erhalten werden. In Benzol als Lösungsmittel wird aus Allylalkohol das 3-Butensäureanhydrid erhalten. Die Einsatzmenge an Palladiumchlorid bezogen auf Allylverbindung beträgt mehr als 5 Mol-% bei Drücken von 100 bis 150 bar und Temperaturen von größer 80 °C.

B. Gabriele et al. beschreiben im Journal of Molecular Catalysis A, 111, 1996, 43-48 die Carbonylierung von Allylalkoholen zu ungesättigten Säuren oder Estern. Die Umsetzung erfolgt in Dimethylacetamid oder Methanol/Dimethylacetamid-Gemischen bei Drücken von 50-100 bar und Temperaturen von größer 80 °C unter Einsatz eines Pd-Katalysators in Beladungen von 2 Mol-% bis 4 Mol-%.

T. Mandai beschreibt in einem Übersichtsartikel im Handbook of Organopalladium Chemistry for Organic Synthesis, Herausgeber E. Negishi, Wiley-VCH, New York, Vol. 2, 2002, 2505-2517 unter anderem die Carbonylierung von Allylverbindungen mit homogenen Palladium-Katalysatoren. Es wird darauf hingewiesen, dass die Carbonylierung allylischer Alkohole drastische Reaktionsbedingungen verlangt. Es wird ebenfalls beschrieben, dass die Carbonylierung von acetylierten Allylalkoholen wie den strukturisomeren Verbindungen (A) und (B) generell schwierig ist. Die Umsetzung gelingt nach der Lehre dieses Dokuments jedoch in Gegenwart katalytischer Mengen von NaBr.

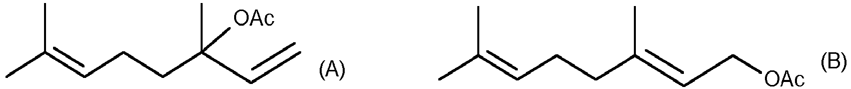

In ACS Catal., 4, 2014, 2977-2989 wird ebenfalls in einem Übersichtsartikel die Carbonylierung von Allylverbindungen mit homogenen Palladium-Katalysatoren beschrieben.

Nach den in der Literatur beschriebenen Verfahren sind zur direkten Carbonylierung von Allylalkoholen hohe Drücken oder Temperaturen oder Katalysatorbeladungen oder der Zusatz von Halogeniden als Additiv, meistens sogar eine Kombination aus wenigstens zwei dieser umsatzsteigenden Maßnahmen nötig, um ausreichend hohe Reaktionsgeschwindigkeiten zu erlangen.

So beschreibt die US 4,801,743 die Carbonylierung von Allylalkohol (2-Propen-1-ol) unter Einsatz eines heterogenen Pd-Katalysators in Abwesenheit von Wasser und in Anwesenheit einer katalytisch wirksamen Menge eines Halogenwasserstoffs, ausgewählt unter HF, HCl, HBr und Hl bei Drücken von größer als 150 bar und Temperaturen von größer 80 °C.

Die EP 0428979 A2 beschreibt die Carbonylierung von allylischen Butenolen und Butenolestern unter Einsatz eines homogenen Rh-Katalysators in Anwesenheit von HBr oder Hl bei CO-Drücken von 1 bis 200 bar und Temperaturen von 10 bis 250 °C.

In den zuvor genannten Dokumenten werden zwar Verfahren zur Carbonylierung von Allylalkoholen zu ihren C1-verlängerten linearen Carbonsäuren oder Carbonsäureestern beschrieben. Nicht beschrieben ist jedoch die Carbonylierung von Allylalkoholen mit terpenartigen Kohlenwasserstoffresten und speziell die Carbonylierung von Linalool zu E/Z-4,8-Dimethyl-3,7-nonadiensäure und von E-Nerolidol oder Farnesol zu E/Z-Homofarnesylsäure. Die in den zuvor genannten Dokumenten beschriebenen Verfahren weisen auch alle einen oder mehrere Nachteile auf, die sie für einen Einsatz zu den besagten Umsetzungen ungeeignet erscheinen lassen. So weisen die in den zuvor genannten Dokumenten beschriebenen Katalysatoren eine zu geringe Aktivität auf oder die Verfahren erfordern zu harsche Reaktionsbedingungen (bzgl. Säure, CO-Druck, Temperatur), um eine effektive, ökonomische Carbonylierung von Allylalkoholen insgesamt und speziell der genannten Allylalkohole mit terpenartigen Kohlenwasserstoffresten zu ermöglichen.

So sind Verfahren, die eine Hochdruckfahrweise erforderlich machen, industriell sehr kostspielig. In vielen der genannten Verfahren ist auch die eingesetzte Katalysatormenge bezogen auf das zu carbonylierende Substrat sehr hoch. Beim Einsatz homogener Katalysatorsysteme, die in der Regel mit dem Produkt ausgetragen werden, ergeben sich somit hohe Kosten durch das Katalysatorrecycling sowie unvermeidbare Katalysatorverluste. Problematisch ist auch der Einsatz von zugesetzten Halogenwasserstoffsäuren und deren Salzen. Aufgrund der hohen Korrosivität dieser Verbindungen ist der Einsatz von Reaktoren aus besonders hochwertigen Stählen erforderlich. Zudem sind derartige Prozessbedingungen für die Umsetzung von E-Nerolidol zu Homofarnesylsäure nicht geeignet, da E-Nerolidol nicht ausreichend stabil gegenüber starken Säuren wie Hl ist.

Verfahren zur Carbonylierung von E-Nerolidol in Gegenwart von homogenen Katalysatoren zu Homofarnesylsäure oder Homofarnesylsäurealkylestern sind prinzipiell bekannt.

Die WO 92/06063 beschreibt ein Verfahren zur Herstellung von ungesättigten Carbonsäuren durch Carbonylierung der entsprechenden allylischen Alkohole, z. B. die Carbonylierung von (*E*)-Nerolidol unter Zusatz katalytischer Mengen von Palladium(II)-chlorid.

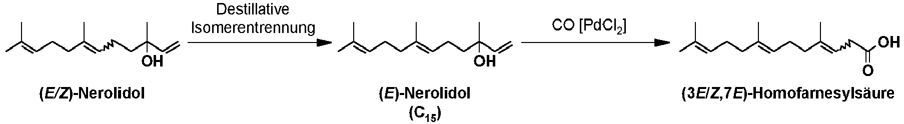

Beschrieben ist weiterhin die Reduktion des so erhaltenen Carbonylierungsprodukts zu Homofarnesol oder Monocyclohomofarnesol und die säurekatalysierte Zyklisierung von Homofarnesol unter Erhalt von 3a,6,6,9a-Tetramethyldodecahydronaphtho-[2,1-b]-furan, einem ambraartigen Duftstoff. Nachteilig an diesem Verfahren ist, dass die Carbonylierungsreaktion bei hohen CO-Drücken von ca. 70 bar stattfindet. Zudem wird eine hohe Katalysatorbeladung von 0,6 Mol-% eingesetzt.

Die EP 0146859 A2 beschreibt ein Verfahren zur Herstellung von 4-substituierten But-3-en-1-carbonsäuren und deren Estern durch Carbonylierung in Anwesenheit oder Abwesenheit eines niederen Alkanols (speziell Methanol) mit Komplexen aus einen Palladiumhalogenid und einem tertiären organischen Phosphin. Die Umsetzung erfolgt bei Drücken von 200 bis 700 bar und Temperaturen von 50 bis 150 °C.

Weiterhin ist bekannt, zur Carbonylierung Ersatzstoffe für CO einzusetzen, die eine drucklose Umsetzung ermöglichen. In WO 2015/061764 und Tetrahedron: Asymmetry, 20, 2009, 1637-1640 wird der Pd-katalysierte Einsatz von N,N-Dimethylformamid-dimethylacetal (DMFDMA) als CO-Substitut in der Umsetzung von E-Nerolidol und anschließende Verseifung in Methanol zu Homofarnesylsäuremethylester beschrieben. Ein solches zweistufiges Verfahren ist jedoch synthetisch aufwändig und auch infolge der eingesetzten CO-Substitute kostspielig, so dass es zur Carbonylierung von Allylalkoholen in einem technischen Maßstab nicht ökonomisch ist.

Es ist weiterhin bekannt, dass sich Allylalkohole in die entsprechenden Acetate überführen lassen und diese Acetate als Substrate in Carbonylierungsreaktionen bei milderen Bedingungen (Drücke z. B. von 30 bar unterhalb von 100 °C) eingesetzt werden können.

In JOC, 53 1988, 3832-3828 wird die Pd- oder Pt-katalysierte cyclisierende Carbonylierung von Zimtsäureacetaten zu 1-Naphtholderivaten beschrieben. Die Reaktion erfolgt in Gegenwart von Acetanhydrid und Triethylamin bei 160 °C und 60 bar CO. Die Zugabe von Acetanhydrid wird mit der Notwendigkeit zur Überführung des intermediär gebildeten 1-Naphthols in das entsprechende Acetat begründet, da freies 1-Naphthol die Cyclocarbonylierung inhibieren würde. Diese Reaktion ist zwar vom Typus her eine Carbonylierung in Gegenwart von Pd und Acetanhydrid, führt jedoch nicht zu Carbonsäurederivaten.

In Tetrahedron Letters, 29, 1988, 4945-4948 wird die Umsetzung von Allylacetaten in Ethanol zu den entsprechenden C1-verlängerten Carbonsäureestern beschrieben. Die Umsetzungen erfolgen bei 30 bis 80 bar CO und 50 bis 80 °C. Die Umsetzung erfolgt jedoch nicht nur in Gegenwart einer Base sondern auch von katalytischen Mengen Bromid-Ionen, wodurch die Reaktion dramatisch beschleunigt werden soll. Es wird in diesem Dokument zudem darauf hingewiesen, dass mit Pd(II)-Katalysatoren, wie Palladium(II)-acetat (Pd(OAc)₂) schlechte Ergebnisse erzielt wurden.

JOC, 58, 1993, 1538-1545 beschreibt die Pd(O)-katalysierte Alkoxycarbonylierung von Allylacetaten. Auch in diesem Dokument wird auf die Notwendigkeit der Anwesenheit von Halogenidionen zur Beschleunigung der Reaktion hingewiesen. Den Allylacetaten selbst wird hingegen eine nur geringe Reaktivität in der Carbonylierung zugeschrieben.

In der Literatur findet sich also speziell keine Vorschrift zur direkten Carbonylierung von Allylalkoholen und speziell von E-Nerolidol zu Homofarnesylsäure (oder Derivaten der Homofarnesylsäure wie den entsprechenden Salzen oder Estern), das effektiv und ökonomisch bei milden Reaktionsbedingungen abläuft. Darunter wird verstanden, dass die Umsetzung bei Drücken im Bereich von höchstens 30 bar CO und/oder bei Temperaturen unterhalb von 100 °C und/oder in Abwesenheit von Halogenverbindungen ausgewählt aus Halogenwasserstoffsäuren und Alkalimetall-, Erdalkalimetall- oder Ammoniumhalogeniden oder einer Kombination von wenigstens zwei dieser Maßnahmen abläuft.

Überraschenderweise wurde gefunden, dass schon die Zugabe katalytischer Mengen an einem Carbonsäureanhydrid, wie Ac₂O (Acetanhydrid) ausreicht, um Allylalkohole bei milden Reaktionsbedingungen und niedrigen Katalysatorbeladungen vollständig zur entsprechenden Carbonsäure zu carbonylieren. Dabei werden unter Zusatz eines nucleophilen Reagenzes wie ein Alkylpyridin, 4-(1-Pyrrolidinyl)pyridin oder ein Dialkylaminopyridin, speziell DMAP (4-Dimethylaminopyridin) besonders vorteilhafte Ergebnisse erzielt. Besonders überraschend reicht auch die Zugabe von katalytischen Mengen von Acylierungsprodukten von Allylalkoholen ohne die Zugabe von Carbonsäureanhydriden aus, um die Reaktion um Größenordnungen zu beschleunigen. Bei diesen Acylierungsprodukten von Allylalkoholen kann es sich auch um Ester von vom Substrat selbst verschiedenen Allylalkoholen mit Carbonsäuren handeln (wie z. B. Allylacetat). Im Lichte des Standes der Technik hätte der Fachmann, wenn er den Einsatz von Acylierungsprodukten des Allylalkohols überhaupt in Betracht gezogen hätte, eine mindestens stöchiometrische Menge an acyliertem Allylalkohol (oder eines Acylierungsmittels) eingesetzt. Dass diese Reaktionsführung so wie beschrieben mit katalytischen Mengen an aktivierendem Reagenz funktioniert, war unbekannt, nicht naheliegend und ist sehr vorteilhaft für die Effizienz des gesamten Prozesses.

Der vorliegenden Erfindung liegt daher die Aufgabe zugrunde, ein Verfahren zur Carbonylierung von Allylalkoholen unter Erhalt von ungesättigten Carbonsäuren oder deren Salzen zur Verfügung zu stellen, bei dem die zuvor genannten Nachteile vermieden werden.

In einer speziellen Ausführung liegt der Erfindung die Aufgabe zugrunde, ein Verfahren zur Carbonylierung von E-Nerolidol zur Verfügung zu stellen, bei dem ein Produktgemisch erhalten wird, welches (3E,7E)-4,8,12-Trimethyltrideca-3,7,11-triensäure (Homofarnesylsäure) und (3Z,7E)-4,8,12-Trimethyltrideca-3,7,11-triensäure oder deren Salze enthält.

Überraschenderweise wurde jetzt gefunden, dass die Carbonylierung von Allylalkoholen und Derivaten des Allylalkohols zu C1-verlängerten ungesättigten Carbonsäuren oder entsprechenden Carbonsäuresalzen bei Temperaturen von höchstens 100 °C durchgeführt werden kann. Vorteilhafterweise wurde gefunden, dass die Carbonylierung von Allylalkoholen und Derivaten des Allylalkohols zu C1-verlängerten ungesättigten Carbonsäuren oder entsprechenden Carbonsäuresalzen bei Temperaturen von höchstens 100 °C und bei niedrigem Druck, z. B. bei einem Druck von höchstens 30 bar durchgeführt werden kann. Vorteilhafterweise wurde zudem gefunden, dass die Carbonylierung von Allylalkoholen und Derivaten des Allylalkohols zu C1-verlängerten ungesättigten Carbonsäuren oder entsprechenden Carbonsäuresalzen bei Temperaturen von höchstens 100 °C bei gleichzeitig niedrigen Katalysatorbeladungen von weniger als 0,3 Mol-% und bei gleichzeitig niedrigem Druck, z. B. bei einem Druck von höchstens 30 bar durchgeführt werden kann. Zudem wurde gefunden, dass die Umsetzung auch ohne Zugabe von beschleunigenden Halogeniden erfolgen kann.

### ZUSAMMENFASSUNG DER ERFINDUNG

Ein erster Gegenstand der Erfindung ist ein Verfahren zur Herstellung einer Zusammensetzung, enthaltend wenigstens eine ungesättigte Carbonsäure der allgemeinen Formel (I) 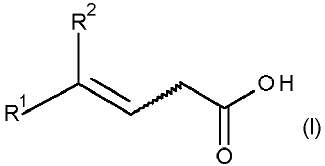 oder eines Salzes davon, worin
- R¹: für Wasserstoff, lineares oder verzweigtes C₁-C₂₄-Alkyl, lineares oder verzweigtes C₂-C₂₄-Alkenyl mit 1, 2, 3 oder mehr als 3 C-C-Doppelbindungen, unsubstituiertes oder mit 1, 2 oder 3 C₁-C₆-Alkylresten substituiertes C₅-C₁₂-Cycloalkyl oder unsubstituiertes oder mit 1, 2 oder 3 C₁-C₆-Alkylresten substituiertes Aryl steht,
- R²: für Wasserstoff, lineares oder verzweigtes C₁-C₂₄-Alkyl oder lineares oder verzweigtes C₂-C₂₄-Alkenyl mit 1, 2, 3 oder mehr als 3 C-C-Doppelbindungen steht, oder
- R¹ und R²: gemeinsam mit dem Kohlenstoffatom, an das sie gebunden sind, für unsubstituiertes C₅-C₇-Cycloalkyl oder für C₅-C₇-Cycloalkyl, das 1, 2 oder 3 lineare oder verzweigte C₁-C₆-Alkylreste trägt, stehen,
bei dem man einen Allylalkohol, ausgewählt unter Verbindungen der allgemeinen Formeln (II.1) und (II.2) 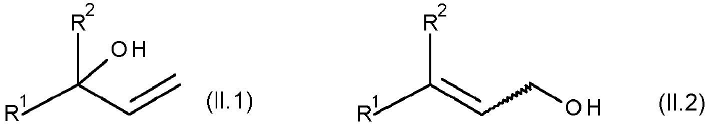 einer Carbonylierung durch Umsetzung mit Kohlenmonoxid in Gegenwart eines Übergangsmetallkatalysators unterzieht, der wenigstens ein Metall der Gruppen 8, 9 oder 10 des Periodensystems der Elemente umfasst, wobei die Umsetzung zusätzlich in Gegenwart wenigstens einer organischen Phosphorverbindung als Ligand und in Gegenwart einer bezogen auf den Allylalkohol unterstöchiometrischen Menge eines Verbindung A) erfolgt, die ausgewählt ist unter Anhydriden aliphatischer C₁-C₁₂-Monocarbonsäuren, Anhydriden aliphatischer C₄-C₂₀-Dicarbonsäuren, Anhydriden cycloaliphatischer C₇-C₂₀-Dicarbonsäuren, Anhydriden aromatischer C₃-C₂₀-Dicarbonsäuren und acylierten Allylalkoholen der Formeln (III.1) und (III.2) 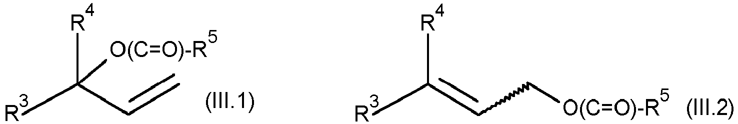 worin
- R³: für Wasserstoff, lineares oder verzweigtes C₁-C₂₄-Alkyl, lineares oder verzweigtes C₂-C₂₄-Alkenyl mit 1, 2, 3 oder mehr als 3 C-C-Doppelbindungen, unsubstituiertes oder mit 1, 2 oder 3 C₁-C₆-Alkylresten substituiertes C₅-C₁₂-Cycloalkyl oder unsubstituiertes oder mit 1, 2 oder 3 C₁-C₆-Alkylresten substituiertes Aryl steht,
- R⁴: für Wasserstoff, lineares oder verzweigtes C₁-C₂₄-Alkyl oder lineares oder verzweigtes C₂-C₂₄-Alkenyl mit 1, 2, 3 oder mehr als 3 C-C-Doppelbindungen steht, oder
- R³ und R⁴: gemeinsam mit dem Kohlenstoffatom, an das sie gebunden sind, für unsubstituiertes C₅-C₇-Cycloalkyl oder für C₅-C₇-Cycloalkyl, das 1, 2 oder 3 lineare oder verzweigte C₁-C₆-Alkylreste trägt, stehen,
- R⁵: für C₁-C₅-Alkyl steht,
und wobei die Umsetzung bei einer Temperatur von höchstens 100 °C erfolgt.

Die Ausführungsform des erfindungsgemäßen Verfahrens, bei der man zur Umsetzung einen Allylalkohol, ausgewählt unter Verbindungen der allgemeinen Formeln (11.1) und (II.2) in Gegenwart einer Verbindung A) einsetzt, wird im Folgenden auch als "Variante 1" bezeichnet.

Eine spezielle Ausführungsform der Variante 1 ist ein Verfahren zur Herstellung einer Zusammensetzung, enthaltend wenigstens eine ungesättigte Carbonsäure der allgemeinen Formel (I) 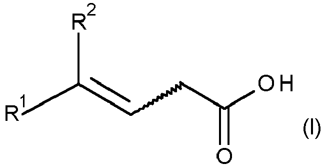 oder eines Salzes davon, worin
- R¹: für Wasserstoff, lineares oder verzweigtes C₁-C₂₄-Alkyl, lineares oder verzweigtes C₂-C₂₄-Alkenyl mit 1, 2, 3 oder mehr als 3 C-C-Doppelbindungen, unsubstituiertes oder mit 1, 2 oder 3 C₁-C₆-Alkylresten substituiertes C₅-C₁₂-Cycloalkyl oder unsubstituiertes oder mit 1, 2 oder 3 C₁-C₆-Alkylresten substituiertes Aryl steht,
- R²: für Wasserstoff, lineares oder verzweigtes C₁-C₂₄-Alkyl oder lineares oder verzweigtes C₂-C₂₄-Alkenyl mit 1, 2, 3 oder mehr als 3 C-C-Doppelbindungen steht, oder
- R¹ und R²: gemeinsam mit dem Kohlenstoffatom, an das sie gebunden sind, für unsubstituiertes C₅-C₇-Cycloalkyl oder für C₅-C₇-Cycloalkyl, das 1, 2 oder 3 lineare oder verzweigte C₁-C₆-Alkylreste trägt, stehen,
bei dem man einen Allylalkohol, ausgewählt unter Verbindungen der allgemeinen Formeln (II.1) und (II.2) 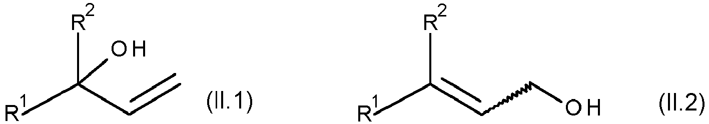 einer Carbonylierung durch Umsetzung mit Kohlenmonoxid in Gegenwart eines Übergangsmetallkatalysators unterzieht, der wenigstens ein Metall der Gruppen 8, 9 oder 10 des Periodensystems der Elemente umfasst, wobei die Umsetzung zusätzlich in Gegenwart wenigstens einer organischen Phosphorverbindung als Ligand und in Gegenwart einer bezogen auf den Allylalkohol unterstöchiometrischen Menge eines Verbindung A) erfolgt, die ausgewählt ist unter Anhydriden aliphatischer C₁-C₁₂-Monocarbonsäuren, Anhydriden aliphatischer C₄-C₂₀-Dicarbonsäuren, Anhydriden cycloaliphatischer C₇-C₂₀-Dicarbonsäuren, Anhydriden aromatischer C₃-C₂₀-Dicarbonsäuren und acylierten Allylalkoholen der Formeln (III.1) und (III.2) 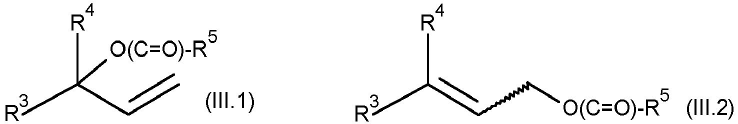 worin
- R³: für Wasserstoff, lineares oder verzweigtes C₁-C₂₄-Alkyl, lineares oder verzweigtes C₂-C₂₄-Alkenyl mit 1, 2, 3 oder mehr als 3 C-C-Doppelbindungen, unsubstituiertes oder mit 1, 2 oder 3 C₁-C₆-Alkylresten substituiertes C₅-C₁₂-Cycloalkyl oder unsubstituiertes oder mit 1, 2 oder 3 C₁-C₆-Alkylresten substituiertes Aryl steht,
- R⁴: für Wasserstoff, lineares oder verzweigtes C₁-C₂₄-Alkyl oder lineares oder verzweigtes C₂-C₂₄-Alkenyl mit 1, 2, 3 oder mehr als 3 C-C-Doppelbindungen steht, oder
- R³ und R⁴: gemeinsam mit dem Kohlenstoffatom, an das sie gebunden sind, für unsubstituiertes C₅-C₇-Cycloalkyl oder für C₅-C₇-Cycloalkyl, das 1, 2 oder 3 lineare oder verzweigte C₁-C₆-Alkylreste trägt, stehen,
- R⁵: für C₁-C₅-Alkyl steht,
und wobei die Umsetzung bei einer Temperatur von höchstens 100 °C und bei einem Druck von höchstens 30 bar erfolgt.

Gegenstand dieser Anmeldung ist nicht eine alternative Ausführungsform, in der zur Carbonylierung ein acylierter Allylalkohol eingesetzt wird. Auch in dieser Variante ist es möglich, die Reaktion unter vorteilhaft milden Reaktionsbedingungen durchzuführen. Die Umsetzung erfolgt nach dieser Variante in Gegenwart von Wasser. Nicht Gegenstand der Erfindung ist ein Verfahren zur Herstellung einer Zusammensetzung, enthaltend wenigstens eine ungesättigte Carbonsäure der allgemeinen Formel (I) 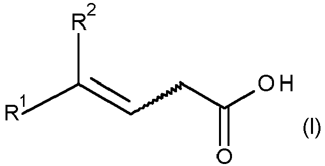 oder eines Salzes davon, worin
- R¹: für Wasserstoff, lineares oder verzweigtes C₁-C₂₄-Alkyl, lineares oder verzweigtes C₂-C₂₄-Alkenyl mit 1, 2, 3 oder mehr als 3 C-C-Doppelbindungen, unsubstituiertes oder mit 1, 2 oder 3 C₁-C₆-Alkylresten substituiertes C₅-C₁₂-Cycloalkyl oder unsubstituiertes oder mit 1, 2 oder 3 C₁-C₆-Alkylresten substituiertes Aryl steht,
- R²: für Wasserstoff, lineares oder verzweigtes C₁-C₂₄-Alkyl oder lineares oder verzweigtes C₂-C₂₄-Alkenyl mit 1, 2, 3 oder mehr als 3 C-C-Doppelbindungen steht, oder
- R¹ und R²: gemeinsam mit dem Kohlenstoffatom, an das sie gebunden sind, für unsubstituiertes C₅-C₇-Cycloalkyl oder für C₅-C₇-Cycloalkyl, das 1, 2 oder 3 lineare oder verzweigte C₁-C₆-Alkylreste trägt, stehen,
bei dem man einen acylierten Allylalkohol, ausgewählt unter Verbindungen der allgemeinen Formeln (IV.1) und (IV.2) 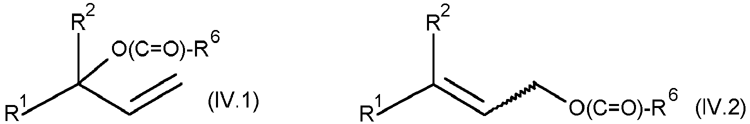 worin R⁶ für C₁-C₅-Alkyl steht,
einer Carbonylierung durch Umsetzung mit Kohlenmonoxid in Gegenwart eines Übergangsmetallkatalysators unterzieht, der wenigstens ein Metall der Gruppen 8, 9 oder 10 des Periodensystems der Elemente umfasst, wobei die Umsetzung zusätzlich in Gegenwart wenigstens einer organischen Phosphorverbindung als Ligand und in Gegenwart von Wasser erfolgt und wobei die Umsetzung bei einer Temperatur von höchstens 100 °C erfolgt.

Die Ausführungsform des nicht erfindungsgemäßen Verfahrens, bei der man zur Umsetzung einen acylierten Allylalkohol, ausgewählt unter Verbindungen der allgemeinen Formeln (IV.1) und (IV.2) einsetzt, wird im Folgenden auch als "Variante 2" bezeichnet.

Eine spezielle Ausführungsform der Variante 2 ist ein Verfahren zur Herstellung einer Zusammensetzung, enthaltend wenigstens eine ungesättigte Carbonsäure der allgemeinen Formel (I) 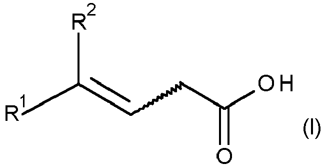 oder eines Salzes davon,
worin
- R¹: für Wasserstoff, lineares oder verzweigtes C₁-C₂₄-Alkyl, lineares oder verzweigtes C₂-C₂₄-Alkenyl mit 1, 2, 3 oder mehr als 3 C-C-Doppelbindungen, unsubstituiertes oder mit 1, 2 oder 3 C₁-C₆-Alkylresten substituiertes C₅-C₁₂-Cycloalkyl oder unsubstituiertes oder mit 1, 2 oder 3 C₁-C₆-Alkylresten substituiertes Aryl steht,
- R²: für Wasserstoff, lineares oder verzweigtes C₁-C₂₄-Alkyl oder lineares oder verzweigtes C₂-C₂₄-Alkenyl mit 1, 2, 3 oder mehr als 3 C-C-Doppelbindungen steht, oder
- R¹ und R²: gemeinsam mit dem Kohlenstoffatom, an das sie gebunden sind, für unsubstituiertes C₅-C₇-Cycloalkyl oder für C₅-C₇-Cycloalkyl, das 1, 2 oder 3 lineare oder verzweigte C₁-C₆-Alkylreste trägt, stehen,

bei dem man einen acylierten Allylalkohol, ausgewählt unter Verbindungen der allgemeinen Formeln (IV.1) und (IV.2) 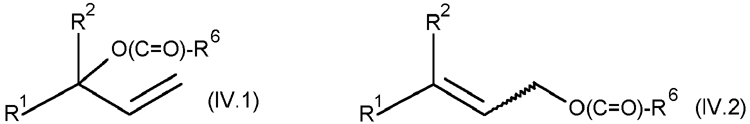 worin R⁶ für C₁-C₅-Alkyl steht,
einer Carbonylierung durch Umsetzung mit Kohlenmonoxid in Gegenwart eines Übergangsmetallkatalysators unterzieht, der wenigstens ein Metall der Gruppen 8, 9 oder 10 des Periodensystems der Elemente umfasst, wobei die Umsetzung zusätzlich in Gegenwart wenigstens einer organischen Phosphorverbindung als Ligand und in Gegenwart von Wasser erfolgt und wobei die Umsetzung bei einer Temperatur von höchstens 100 °C und bei einem Druck von höchstens 30 bar erfolgt.

Eine alternative Ausführungsform betrifft Verfahren zur Herstellung einer Zusammensetzung, enthaltend wenigstens eine ungesättigte Carbonsäure der allgemeinen Formel (I) 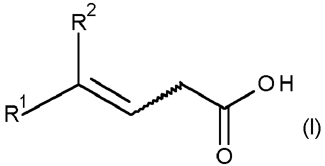 oder eines Salzes davon,
worin
- R¹: für Wasserstoff, lineares oder verzweigtes C₁-C₂₄-Alkyl, lineares oder verzweigtes C₂-C₂₄-Alkenyl mit 1, 2, 3 oder mehr als 3 C-C-Doppelbindungen, unsubstituiertes oder mit 1, 2 oder 3 C₁-C₆-Alkylresten substituiertes C₅-C₁₂-Cycloalkyl oder unsubstituiertes oder mit 1, 2 oder 3 C₁-C₆-Alkylresten substituiertes Aryl steht,
- R²: für Wasserstoff, lineares oder verzweigtes C₁-C₂₄-Alkyl oder lineares oder verzweigtes C₂-C₂₄-Alkenyl mit 1, 2, 3 oder mehr als 3 C-C-Doppelbindungen steht, oder
- R¹ und R²: gemeinsam mit dem Kohlenstoffatom, an das sie gebunden sind, für unsubstituiertes C₅-C₇-Cycloalkyl oder für C₅-C₇-Cycloalkyl, das 1, 2 oder 3 lineare oder verzweigte C₁-C₆-Alkylreste trägt, stehen,
bei dem man einen Allylalkohol, ausgewählt unter Verbindungen der allgemeinen Formeln (II.1) und (II.2) 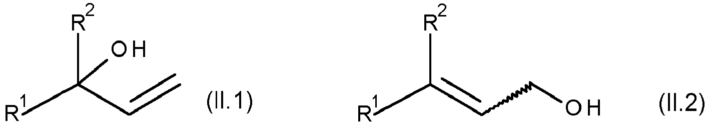 einer Carbonylierung durch Umsetzung mit Kohlenmonoxid in Gegenwart eines Übergangsmetallkatalysators unterzieht, der wenigstens ein Metall der Gruppen 8, 9 oder 10 des Periodensystems der Elemente umfasst, wobei die Umsetzung zusätzlich in Gegenwart wenigstens einer organischen Phosphorverbindung als Ligand und in Gegenwart einer bezogen auf den Allylalkohol unterstöchiometrischen Menge einer Verbindung B) 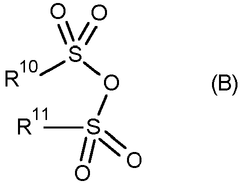 worin
- R¹⁰ und R¹¹,: unabhängig voneinander, für C₁-C₆-Alkyl, C₁-C₆-Fluoralkyl, Phenyl, das unsubstituiert ist oder mit einem Substituenten ausgewählt aus Brom, Nitro und C₁-C₄-Alkyl ist, stehen;
und wobei die Umsetzung bei einer Temperatur von höchstens 100 °C erfolgt. Speziell erfolgt die Umsetzung bei einer Temperatur von höchstens 100 °C und einem Druck von höchstens 30 bar.

Auch in dieser Variante ist es möglich, die Reaktion unter vorteilhaft milden Reaktionsbedingungen durchzuführen. Die Ausführungsform des erfindungsgemäßen Verfahrens, bei der man zur Umsetzung einen Allylalkohol, ausgewählt unter Verbindungen der allgemeinen Formeln (II.1) und (II.2) in Gegenwart einer Verbindung B) einsetzt, wird im Folgenden auch als "Variante 3" bezeichnet.

Ein weiterer Gegenstand der Erfindung ist ein Verfahren zur Herstellung von (-)-Ambrox (VIII) 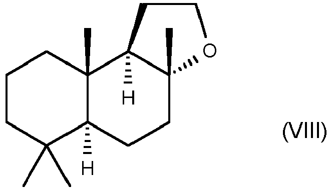 bei dem man
a1) nach einem erfindungsgemäßen Verfahren wie zuvor definiert ein Gemisch aus (3E,7E)-4,8,12-Trimethyltrideca-3,7,11-triensäure und (3Z,7E)-4,8,12-Trimethyltrideca-3,7,11-triensäure bereitstellt;
b1) das Gemisch aus (3E,7E)-4,8,12-Trimethyltrideca-3,7,11-triensäure und (3Z,7E)-4,8,12-Trimethyltrideca-3,7,11-triensäure einer Trennung unter Erhalt von (3E,7E)-4,8,12-Trimethyltrideca-3,7,11-triensäure unterzieht;
c1) die (3E,7E)-4,8,12-Trimethyltrideca-3,7,11-triensäure einer Reduktion unter Erhalt von (3E,7E)-Homofarnesol (VI) unterzieht;
d1) das (3E,7E)-Homofarnesol (VI) einer Zyklisierung unter Erhalt von (-)-Ambrox (VIII) unterzieht;
   oder
a2) nach einem erfindungsgemäßen Verfahren wie zuvor definiert ein Gemisch aus (3E,7E)-4,8,12-Trimethyltrideca-3,7,11-triensäure und (3Z,7E)-4,8,12-Trimethyltrideca-3,7,11-triensäure bereitstellt;
b2) das Gemisch aus (3E,7E)-4,8,12-Trimethyltrideca-3,7,11-triensäure und (3Z,7E)-4,8,12-Trimethyltrideca-3,7,11-triensäure einer Trennung unter Erhalt von (3E,7E)-4,8,12-Trimethyltrideca-3,7,11-triensäure unterzieht;
c2) die (3E,7E)-4,8,12-Trimethyltrideca-3,7,11-triensäure einer Zyklisierung unter Erhalt von Sclareolid (VII) unterzieht;
d2) das Sclareolid (VII) einer Reduktion unter Erhalt von (-)-Ambrox (VIII) unterzieht.

### BESCHREIBUNG DER ERFINDUNG

Die erfindungsgemäßen Verfahren zur Herstellung einer Zusammensetzung, enthaltend wenigstens eine ungesättigte Carbonsäure der allgemeinen Formel (I) weisen die folgenden Vorteile auf:
- Variante 1 des Verfahrens ermöglicht die effektive und ökonomische Umsetzung von Allylalkoholen zu ihren C1-verlängerten ungesättigten Carbonsäuren oder entsprechenden Carbonsäuresalzen. Dabei sind deutlich mildere Reaktionsbedingungen (bei gleichzeitig sehr niedrigen Katalysatorbeladungen) möglich als bei den aus dem Stand der Technik bekannten Verfahren. Speziell gelingt die Umsetzung bei niedrigerer Temperatur und/oder einem geringeren Druck und/oder einer geringeren Katalysatorbeladung als bei den bekannten Verfahren.
- Besonders vorteilhaft ist, dass in Variante 1 des erfindungsgemäßen Verfahrens die Umsetzung in Gegenwart katalytischer (d. h. unterstöchiometrischer) Mengen an Anhydrid ausreicht, um die allylischen Alkohole bei milden Reaktionsbedingungen und niedrigen Katalysatorbeladungen vollständig zu den entsprechenden Carbonsäuren zu carbonylieren. Besonders überraschend reicht auch die Zugabe von katalytischen Mengen anderer Anhydridbildner, wie Allylacetat (Propenylacetat) oder hiervon verschiedenen Acylierungsprodukten von Allylalkoholen aus, um die Reaktion deutlich zu beschleunigen. Die Zugabe von zusätzlichem Anhydrid ist dabei nicht erforderlich. Ohne an eine Theorie gebunden sein zu wollen, wird angenommen, dass dies darauf zurückzuführen ist, dass unter den Reaktionsbedingungen zunächst eine Carbonylierung des zugesetzten acylierten Allylalkohols (z. B. Allylacetat CH₂=CHCH₂OAc) zu einer Anhydrid-Zwischenstufe (z. B. CH₂=CHCH₂C(O)OAc) erfolgt, das in situ gebildete Anhydrid den eingesetzten allylischen Alkohol (z. B. Nerolidol) unter Eliminierung von Carbonsäure (z. B. CH₂=CHCH₂CO₂H) aktiviert, wobei gleichzeitig neuer acylierter Allylalkohol (z. B. Nerolidylacetat) generiert wird, der wiederum erneut zum entsprechenden Anhydridintermediat (gemischtes Anhydrid aus Essigsäure und Homofarnesylsäure) carbonyliert werden kann.
- Die Möglichkeit zum Einsatz katalytischer Mengen eines nucleophilen Reagenzes in Variante 1 des Verfahrens ist sehr vorteilhaft für die Effizienz des gesamten Prozesses.
- Das erfindungsgemäße Verfahren nach Variante 1 ermöglicht die Carbonylierung von Allylalkoholen unter Erhalt der entsprechenden Carbonsäuren oder Carbonsäuresalze in einer einzigen Reaktionsstufe, d.h. ohne die Isolierung eines Zwischenprodukts. So ist es beispielsweise nicht erforderlich, in einem ersten Reaktionsschritt unter Verwendung stöchiometrischer Mengen an Acylierungsmittel den eingesetzten Allylalkohol zu aktivieren.
- Enthalten die zur Carbonylierung eingesetzten Allylalkohole weitere innenliegende Doppelbindungen, so werden diese im Wesentlichen weder carbonyliert noch isomerisiert.
- Die nicht erfindungsgemäße alternative Variante 2 des Verfahrens beruht auf der Carbonylierung von acylierten Allylalkoholen zu ihren C1-verlängerten ungesättigten Carbonsäuren in Gegenwart von Wasser unter milden Reaktionsbedingungen.
- Die Verfahren nach Variante 1 und 2 ermöglichen, auf den Einsatz von zugesetzten Halogenwasserstoffsäuren und deren Salze zu verzichten.
- Das Verfahren nach Variante 1 ermöglicht speziell die Carbonylierung von Allylalkoholen mit terpenartigen Kohlenwasserstoffresten und speziell die Carbonylierung von Linalool zu E/Z-4,8-Dimethyl-3,7-nonadiensäure und von E-Nerolidol oder Farnesol zu E/Z-Homofarnesylsäure bei milden Reaktionsbedingungen.
- Mit dem Verfahren nach Variante 1 gelingt in vorteilhafter Weise die Carbonylierung von E-Nerolidol unter Erhalt eines Reaktionsgemischs aus (3E,7E)-4,8,12-trimethyltrideca-3,7,11-triensäure (Homofarnesylsäure) und (3Z,7E)-4,8,12-trimethyltrideca-3,7,11-triensäure oder den Salzen davon.

In den Verbindungen der allgemeinen Formeln (I), (II.2), (III.2) und (IV.2) 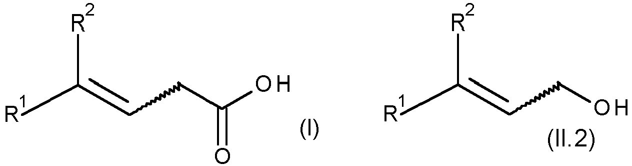 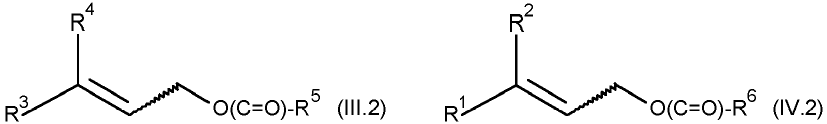 soll die geschlängelte Bindung andeuten, dass es sich jeweils um das reine Z-Isomer, das reine E-Isomer oder ein beliebiges E-/Z-Gemisch handeln kann. Es versteht sich für den Fachmann von selbst, dass in den Verbindungen der allgemeinen Formeln (I), (II.2), (und (IV.2) E- und Z-Isomere nur vorliegen können, wenn zwei verschiedene Substituenten R1 und R2 an der Doppelbindung gebunden sind. In den Verbindungen der allgemeinen Formel (III.2) können E- und Z-Isomere nur vorliegen, wenn zwei verschiedene Substituenten R3 und R4 an der Doppelbindung gebunden sind. Die stereochemische Konfiguration an der Doppelbindung wird mit Hilfe der Cahn-Ingold-Prelog Regeln ermittelt (siehe E/Z-notation, https://en.wikipedia.org/wiki/E-Z_notation).

Im Rahmen der vorliegenden Erfindung wird das E/Z-Gemisch der allgemeinen Formel (I) auch als E/Z-Säuregemisch der Formel (I) bezeichnet. In dem E/Z-Säuregemisch der allgemeinen Formel (I) weisen R¹ und R² unterschiedliche Bedeutungen auf. Das E/Z-Säuregemisch der Formel (I) enthält das 3-(E)-Isomer der ungesättigten Carbonsäure der Formel I und das 3-(Z)-Isomer der ungesättigten Carbonsäure der Formel I. Im Folgenden wird das 3-(E)-Isomer der ungesättigten Carbonsäure der Formel I als 3-(E)-Isomer der Formel (I-E) bezeichnet und das 3-(Z)-Isomer der ungesättigten Carbonsäure als 3-(Z)-Isomer der Formel (I-Z) bezeichnet.

In den allgemeinen Formeln der Verbindungen I-Z und I-E 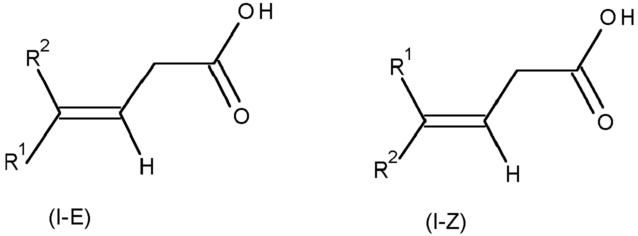 hat der Substituent R¹ eine höhere Priorität nach IUPAC (International Union of Pure and Applied Chemistry, Internationale Union für reine und angewandte Chemie) als der Substituent R². Nach IUPAC erfolgt die Prioritätsreihenfolge der Substituenten gemäß der Cahn-Ingold-Prelog-Regel.

Es wird allgemein angenommen, dass die Übergangsmetall-katalysierte und speziell die Pd-katalysierte Carbonylierung über einen pi-Allyl-Komplex verläuft, der es ermöglicht, dass sowohl ausgehend von den Verbindungen (II.1) als auch von den Verbindungen (II.2) jeweils Reaktionsprodukte erhalten werden können, die die Verbindungen (I) enthalten.

Bevorzugt erfolgt die Carbonylierung nicht in Gegenwart einer zugesetzten Halogenwasserstoffsäure und nicht in Gegenwart eines zugesetzten Alkalimetall-, Erdalkalimetall- oder Ammoniumhalogenids. Dabei umfasst der Ausdruck "Ammoniumsalz" sowohl Salze, die sich von NH₄⁺ ableiten, als auch Mono-, Di-, Tri- und Tetraorganylammoniumsalze. Speziell erfolgt die Carbonylierung nicht in Gegenwart eines zugesetzten Alkylhalogenids, wie NaCl, NaBr, KCl, KBr, LiCI, LiBr, etc.

Bevorzugt beträgt der Halogenidgehalt des Reaktionsgemischs der Carbonylierung nicht mehr als 2 Mol-%, bevorzugt nicht mehr als 1 Mol-%, bezogen auf den Gehalt an Allylalkohol der allgemeinen Formeln (II.1) und (II.2).

Im Rahmen der vorliegenden Erfindung werden die Begriffe "acylierter Allylalkohol", "Carbonsäureallylester", "Acylierungsprodukt von Allylalkohol" und "Acylierungsprodukt eines Allylalkohols" synonym verwendet.

Der Ausdruck "aliphatisch" umfasst im Sinne der vorliegenden Erfindung Kohlenwasserstoffreste, in denen die C-Atome in geraden oder verzweigten Ketten angeordnet sind.

Der Ausdruck "Alkyl" sowie alle Alkylteile in Alkoxy, Alkylamino und Dialkylamino umfasst im Sinne der vorliegenden Erfindung gesättigte, lineare oder verzweigte Kohlenwasserstoffreste mit 1 bis 4 ("C₁-C₄-Alkyl"), 1 bis 6 ("C₁-C₆-Alkyl"), 1 bis 10 ("C₁-C₁₀-Alkyl"), 1 bis 20 ("C₁-C₂₀-Alkyl") oder 1 bis 24 ("C₁-C₂₄-Alkyl") Kohlenstoffatomen. Beispiele für lineares oder verzweigtes C₁-C₄-Alkyl sind Methyl, Ethyl, n-Propyl, 1-Methylethyl, n-Butyl, 1-Methyl-propyl, 2-Methylpropyl, 1,1-Dimethylethyl. Beispiele für lineares oder verzweigtes C₁-C₆-Alkyl sind zusätzlich zu den für C₁-C₄-Alkyl genannten Bedeutungen n-Pentyl, 1-Methylbutyl, 2-Methylbutyl, 3-Methylbutyl, 2,2-Di-methylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl. Beispiele für lineares oder verzweigtes C₁-C₁₀-Alkyl sind zusätzlich zu den für C₁-C₆-Alkyl genannten Bedeutungen Heptyl, Octyl, Nonyl, Decyl und Stellungsisomere davon. Beispiele für C₁-C₂₀-Alkyl sind zusätzlich zu den für C₁-C₁₀-Alkyl genannten Bedeutungen Undecyl, Dodecyl, Tridecyl, Tetradecyl, Pentadecyl, Hexadecyl, Heptadecyl, Octadecyl, Nonadecyl, Eicosyl, Heneicosyl, Docosyl, Tricosyl, Tetracosyl, Pentacosyl, Hexacosyl, Heptacosyl, Octacosyl, Nonacosyl, Eicosyl und Stellungsisomere davon.

Der Ausdruck "Alkenyl" umfasst im Rahmen der vorliegenden Erfindung ungesättigte, lineare oder verzweigte Kohlenwasserstoffreste mit 2 bis 4 (C₂-C₄-Alkenyl), bis 6, bis 8, bis 10, bis 16, bis 20 oder bis 24 Kohlenstoffatomen und einer, zwei, drei oder mehr als drei Doppelbindungen in beliebiger Position. Beispiele für lineares oder verzweigtes C₂-C₂₄-Alkenyl mit einer Doppelbindung sind Vinyl, Allyl (2-Propen-1-yl), 1-Methylprop-2-en-1-yl, 2-Buten-1-yl, 3-Buten-1-yl, n-Pentenyl, n-Hexenyl, n-Heptenyln-Octenyl, n-Nonenyl, n-Decenyl, n-Undecenyl, n-Dodecenyl, n-Tridecenyl, n-Tetradecenyl, n-Pentadecenyl, n-Hexadecenyl, n-Heptadecenyl, n-Octadecenyl, Oleyl, n-Nonadecenyl, n-Eicosenyl, n-Heneicosenyl, n-Docosenyl, n-Tricosenyl, n-Tetracosenyl und deren Konstitutionsisomeren. Beispiele für lineares oder verzweigtes C₄-C₂₄-Alkenyl mit zwei oder drei Doppelbindungen in beliebiger Position sind n-Butadienyl, n-Pentadienyl, n-Hexadienyl, n-Heptadienyl, n-Octadienyl, n-Octatrienyl, n-Nonadienyl, n-Nonatrienyl, n-Decadienyl, n-Decatrienyl, n-Undecadienyl, n-Undecatrienyl, n-Dodecadienyl, n-Dodecatrienyl, n-Tridecadienyl, n-Tridecatrienyl, n-Tetradecadienyl n-Tetradecatrienyl, n-Pentadecadienyl, n-Pentadecatrienyl, n-Hexadecadienyl, n-Hexadecatrienyl, n-Heptadecadienyl, n-Heptadecatrienyl, n-Octadecadienyl, n-Octadecatrienyl, n-Nonadecadienyl, n-Nonadecatrienyl, n-Eicosadienyl, n-Eicosatrienyl, n-Heneicosadienyl, n-Heneicosatrienyl, n-Docosadienyl, n-Docosatrienyl, n-Tricosadienyl, n-Tricosatrienyl, n-Tetracosadienyl, n-Tetracosatrienyl, Linolenyl und deren Konstitutionsisomeren. Jede Doppelbindung in C₂-C₂₄-Alkenyl kann (soweit nichts anderes angegeben ist) jeweils unabhängig voneinander in der E- als auch in Z-Konfiguration vorliegen.

Der Ausdruck "Cycloalkyl" umfasst im Sinne der vorliegenden Erfindung monozyklische, gesättigte Kohlenwaserstoffreste mit 3 bis 8 ("C₃-C₈-Cycloalkyl"), vorzugsweise 5 bis 7 Kohlenstoffringgliedern ("C₅-C₇-cycloalkyl"). Beispiele für C₃-C₈-Cycloalkyl sind Cyclopropyl, Cyclobutyl, Cyclopentyl, Cyclohexyl, Cycloheptyl und Cyclooctyl.

Der Ausdruck "Heterocyclyl" (auch als Heterocycloalkyl bezeichnet) umfasst im Sinne der vorliegenden Erfindung monozyklische, gesättigte zykloaliphatische Gruppen mit im Allgemeinen 5 bis 8 Ringatomen, vorzugsweise 5 oder 6 Ringatomen, in denen 1 oder 2 der Ringkohlenstoffatome durch Heteroatome oder heteroatomhaltige Gruppen, ausgewählt unter Sauerstoff, Stickstoff, NH und N(C₁-C₄-Alkyl), ersetzt sind. Beispiele für solche heterocycloaliphatischen Gruppen sind Pyrrolidinyl, Piperidinyl, Tetrahydrofuranyl und Tetrahydropyranyl.

Der Ausdruck "Aryl" umfasst im Sinne der vorliegenden Erfindung ein ein-, zwei- oder dreikerniges aromatisches Ringsystem, enthaltend 6 bis 20 Kohlenstoffringglieder. Beispiele für unsubstituiertes C₆-C₁₀-Aryl sind Phenyl und Naphthyl. Beispiele für C₆-C₁₄-Aryl sind Phenyl, Naphthyl, Anthracenyl und Phenanthrenyl. Substituierte Arylgruppen tragen im Allgemeinen 1, 2, 3, 4 oder 5 gleiche oder verschiedene, bevorzugt 1, 2 oder 3 Substituenten. Geeignete Substituenten sind insbesondere ausgewählt unter Alkyl, Cycloalkyl, Aryl, Alkoxy, Dialkylamino. Beispiele für Aryl, das 1, 2 oder 3 unter C₁-C₆-Alkyl ausgewählte Substituenten trägt sind Tolyl, Xylyl und Mesityl.

Der Ausdruck "Hetaryl" umfasst im Sinne der vorliegenden Erfindung einen fünf- bis sechsgliedriger aromatischen Heteromonocyclus, enthaltend ein, zwei, drei oder vier Heteroatome aus der Gruppe Sauerstoff, Stickstoff oder Schwefel, und 9- oder 10-gliedrige aromatische Heterobicylcen, z. B. C-gebundenes 5-gliedriges Heteroaryl, enthaltend ein bis drei Stickstoffatome oder ein oder zwei Stickstoffatome und ein Schwefel- oder Sauerstoffatom als Ringglieder wie Furyl, Thienyl, Pyrrolyl, Isoxazolyl, Isothiazolyl, Pyrazolyl, Oxazolyl, Thiazolyl, Imidazolyl, 4-Imidazolyl, 1,2,4-Oxadiazolyl, 1,2,4-Thiadiazolyl, 1,2,4-Triazolyl, 1,3,4-Oxadiazolyl; über Stickstoff gebundenes 5-gliedriges Heteroaryl, enthaltend ein bis drei Stickstoffatome als Ringglieder wie Pyrrol-1-yl, Pyrazol-1-yl, Imidazol-1-yl, 1,2,3-Triazol-1-yl und 1,2,4-Triazol-1-yl; 6-gliedriges Heteroaryl, enthaltend ein bis drei Stickstoffatome als Ringglieder wie Pyridinyl, Pyridazinyl, Pyrimidinyl, Pyrazinyl, 1,3,5-Triazinyl und 1,2,4-Triazinyl.

Halogen steht für Fluor, Chlor, Brom, oder lod. Halogenid steht für Fluorid, Chlorid, Bromid oder lodid.

Alkalimetalle entsprechen den Elementen der Gruppe 1 des Periodensystems der Elemente nach IUPAC, beispielsweise Lithium, Natrium, Kalium, Rubidium oder Cäsium, vorzugsweise Lithium, Natrium oder Kalium, insbesondere Natrium oder Kalium.

Erdalkalimetalle entsprechen den Elementen der Gruppe 2 des Periodensystems der Elemente nach IUPAC, beispielsweise Beryllium, Magnesium, Calcium, Strontium oder Barium, vorzugsweise Magnesium oder Calcium.

Die Gruppe 8 des Periodensystems der Elemente nach IUPAC umfasst u.a. Eisen, Ruthenium und Osmium. Die Gruppe 9 des Periodensystems der Elemente nach IUPAC umfasst u.a. Cobalt, Rhodium, Iridium. Die Gruppe 10 des Periodensystems der Elemente nach IUPAC umfasst u.a. Nickel, Palladium und Platin.

Der Ausdruck "Ammoniumsalze" umfasst im Rahmen der vorliegenden Erfindung sowohl Salze, die sich von NH₄⁺ ableiten, als auch Mono-, Di-, Tri- und Tetraorganylammoniumsalze. Die an den Ammonium-Stickstoff gebundenen Reste sind in der Regeljeweils unabhängig ausgewählt aus Wasserstoff und aliphatischen, alicyclischen und aromatischen Kohlenwasserstoffgruppen. Vorzugsweise sind die an den Ammonium-Stickstoff gebundenen Reste jeweils unabhängig ausgewählt aus Wasserstoff und aliphatischen Resten, speziell ausgewählt aus Wasserstoff und C₁-C₂₀-Alkyl.

Der Ausdruck "Acyl" umfasst im Sinne der vorliegenden Erfindung Alkanoyl- oder Aroylgruppen mit im Allgemeinen 1 bis 11, vorzugsweise 2 bis 8 Kohlenstoffatomen, beispielsweise die Formyl, Acetyl-, Propionyl-, Butyryl-, Pentanoyl-, Benzoyl- oder Naphthoyl-Gruppe.

Die Acetylierung im Sinne der vorliegenden Erfindung ist der Austausch von Wasserstoff durch eine Acetylgruppe (-C(=O)-CH₃).

Soweit im Folgenden nichts genauer angegeben ist, bezeichnen die allgemeinen Formeln (I), (II.2), (III.2) und (IV.2) E/Z-Mischungen jedweder Zusammensetzung und die reinen Konfigurationsisomere. Weiterhin bezeichnen die allgemeinen Formeln (I) (II.2), (III.2) und (IV.2) alle Stereoisomere in reiner Form sowie racemische und optisch aktive Mischungen der Verbindungen der Formeln (II.2), (III.2) und (IV.2).

"Stereoisomere" sind Verbindungen gleicher Konstitution aber unterschiedlicher Atomanordnung im dreidimensionalen Raum.

"Enantiomere" sind Stereoisomere, die sich zueinander wie Bild zu Spiegelbild verhalten.

Der Begriff "terpenartige Kohlenwasserstoffreste" umfasst im Rahmen der vorliegenden Erfindung Kohlenwasserstoffreste, die sich formal von ein, zwei, drei oder mehr als drei Isopreneinheiten ableiten. Die terpenartigen Kohlenwasserstoffreste können dabei noch Doppelbindungen aufweisen oder vollständig gesättigt sein. Die Doppelbindungen können dabei beliebige Positionen einnehmen, wobei kumulierte Doppelbindungen ausgeschlossen sind.

Der Begriff "Farnesol (3,7,11-Trimethyl-2,6,10-dodecatrien-1-ol)" umfasst im Rahmen der vorliegenden Erfindung das (2E,6E)-, (2Z,6Z)-, (2Z,6E)- und das (2E,6Z)-Isomer sowie Mischungen von zwei, drei oder sämtlichen der genannten Isomeren des Farnesols. Farnesol ist käuflich erhältlich.

Nerolidol besitzt ein Chiralitätszentrum und kann als *E*/*Z*-Gemisch vorliegen, es gibt also vier Stereoisomere. Der Begriff "Nerolidol (3,7,11-Trimethyl-1,6,10-dodecatrien-3-ol)" umfasst im Rahmen der vorliegenden Erfindung das (3*S*),(6*Z*)-Isomer, (3*R*),(6*Z*)-Isomer, (3*S*),(6,*E*)-Isomer, (3*R*),(6*E*)-Isomer sowie beliebige Mischungen davon. Nerolidol ist käuflich erhältlich. Nerolidylacetat (3,7,11-Trimethyl-1,6,10-octatrienylacetat) ist das Acetylierungsprodukt von Nerolidol. Es ist ebenfalls käuflich.

Ein anderer Name für Homofarnesylsäure ist 4,8,12,-Trimethyltrideca-3,7,11-triensäure.

Eine erste Ausführungsform der vorliegenden Erfindung (Variante 1) betrifft die Carbonylierung eines unter Verbindungen der Formeln (11.1) und (II.2) ausgewählten allylischen Alkohols (Allylalkohols) zur entsprechenden C1-verlängerten ungesättigten Carbonsäuren der Formel (I).

Die Ausgangsverbindungen der Formel (II.1) sind käuflich erhältlich oder können beispielsweise aus den entsprechenden Carbonylverbindungen R¹-CO-R² und Vinylmagnesiumhalogeniden hergestellt werden. Die Verbindungen der Formel (II.1) können auch aus der entsprechenden Carbonylverbindung und Acetyliden wie Natriumacetylid und anschließende partielle Hydrierung der Ethinylgruppe zur Vinylgruppe hergestellt werden.

Im Rahmen des erfindungsgemäßen Verfahrens bevorzugte Allylalkohole der Formeln (II.1) und (II.2) sind solche, worin die Reste R¹ und R² gleich oder verschieden sind und für Wasserstoff, lineares oder verzweigtes C₁-C₁₆-Alkyl, lineares oder verzweigtes C₂-C₁₆-Alkenyl mit einer oder zwei nicht konjugierten Doppelbindungen stehen oder R¹ und R² zusammen mit dem Kohlenstoffatom, an das sie gebunden sind, für unsubstituiertes C₅-C₇-Cycloalkyl oder C₅-C₇-Cycloalkyl, das 1, 2 oder 3 lineare oder verzweigte C₁-C₆-Alkylreste trägt, stehen.

Insbesondere bevorzugt werden Verbindungen der Formel (II.1) und (II.2) mit terpenartigen Kohlenwasserstoffresten. R¹ steht vorzugsweise für lineares oder verzweigtes C₆-C₁₆-Alkyl oder lineares oder verzweigtes C₆-C₁₆-Alkenyl mit einer oder zwei nicht konjugierten Doppelbindungen. R² steht vorzugsweise für Wasserstoff oder C₁-C₄-Alkyl, speziell C₁-C₂-Alkyl. Erfindungsgemäß besonders bevorzugte Allylalkohole der Formel (II.1) sind 3-Methyl-1-penten-3-ol, 1-Hepten-3-ol, 1-Vinylcyclohexanol, Linalool und Nerolidol, insbesondere Linalool und Nerolidol, speziell 6*E*-Nerolidol. Erfindungsgemäß besonders bevorzugte Allylalkohole der Formel (II.2) sind Geraniol und Farnesol.

Sofern die Verbindungen der Formeln (II.1) und (II.2) ein oder mehrere Asymmetriezentren aufweisen, können auch Enantiomerengemische oder Diastereomerengemische eingesetzt werden. Das erfindungsgemäße Verfahren kann mit einer Mischung der Verbindungen der Formeln (II.1) und (II.2) durchgeführt werden.

Erfindungsgemäß wird das Verfahren nach der Variante 1, bei der man einen Allylalkohol, ausgewählt unter Verbindungen der allgemeinen Formeln (II.1) und (II.2) zur Reaktion einsetzt, in Gegenwart einer Verbindung A) ausgeführt. Als Verbindungen A) kommen vorzugsweise die Anhydride von linearen oder verzweigten einbasigen C₁-C₁₂-Alkancarbonsäuren in Betracht. Beispiele solcher Anhydride sind Acetanhydrid (Essigsäureanhydrid), Propionsäureanhydrid, Isopropionsäureanhydrid, Buttersäureanhydrid, n-Valeriansäureanhydrid, das gemischte Anhydrid von Ameisensäure und Essigsäure und dergleichen. Insbesondere bevorzugt sind die symmetrischen Anhydride der Alkanmonocarbonsäuren mit bis zu 5 Kohlenstoffatomen. Ebenfalls geeignet sind die Anhydride von linearen oder verzweigten einbasigen C₃-C₁₂-Alkencarbonsäuren. Beispiele hierfür sind (Meth)acrylsäureanhydrid, Crotonsäureanhydrid und Isocrotonsäureanhydrid. Ebenfalls geeignet sind die Anhydride von linearen oder verzweigten zweibasigen C₄-C₂₀-Alkancarbonsäuren, z. B. das Anhydrid der Bernsteinsäure, Glutarsäure, Adipinsäure, Pimelinsäure, Korksäure, Azelainsäure, Sebazinsäure, Undecandisäure, Dodecandisäure, Brassylsäure, Tetradecandisäure, Pentadecandisäure, Hexadecandisäure, Heptadecandisäure, Octadecandisäure. Ebenfalls geeignet sind die Anhydride von linearen oder verzweigten zweibasigen C₄-C₂₀-Alkencarbonsäuren, z. B. Maleinsäureanhydrid oder Itakonsäureanhydrid. Ebenfalls geeignet sind die Anhydride von cycloaliphatischen C₇-C₂₀-Dicarbonsäuren wie Cyclopentandicarbonsäureanhydrid oder Cyclohexancarbonsäureanhydrid. Ebenfalls geeignet sind die Anhydride von aromatischen C₃-C₂₀-Dicarbonsäuren wie Phthalsäureanhydrid, 1,8-Naphthalincarbonsäureanhydrid oder 2,3-Naphthalincarbonsäureanhydrid. Besonders bevorzugt werden Acetanhydrid, Propionsäureanhydrid, Isopropionsäureanhydrid, Buttersäureanhydrid, Bernsteinsäureanhydrid, Maleinsäureanhydrid oder Phthalsäureanhydrideingesetzt, speziell Acetanhydrid.

Geeignete Verbindungen A) sind ebenfalls die Ester der Formeln (III.1) und (III.2). Bei dem erfindungsgemäßen Verfahren kommt es nicht darauf an, ob die Doppelbindung endständig angeordnet ist (Verbindung der Formeln (111.1)) oder ob die Doppelbindung innenständig angeordnet ist (Verbindung der Formel (III.2)). Daher kann auch ein Gemisch an Verbindungen der Formeln (III.1) und (III.2) eingesetzt werden. In der Regel wird jedoch kein Gemisch der Verbindungen der Formeln (III.1) und (III.2) eingesetzt.

Bevorzugt sind Verbindungen der Formeln (III.1) und (III.2), worin die Reste R³ und R⁴ gleich oder verschieden sind und für Wasserstoff, lineares oder verzweigtes C₁-C₁₆-Alkyl, lineares oder verzweigtes C₂-C₁₆-Alkenyl mit einer oder zwei nicht konjugierten Doppelbindungen stehen oder R³ und R⁴ zusammen mit dem Kohlenstoffatom, an das sie gebunden sind, für unsubstituiertes C₅-C₇-Cycloalkyl oder C₅-C₇-Cycloalkyl, das 1, 2 oder 3 lineare oder verzweigte C₁-C₆-Alkylreste trägt, stehen. Alternativ stehen in den Verbindungen der Formeln (III.1) und (III.2) R³ und R⁴ gemeinsam mit dem Kohlenstoffatom, an das sie gebunden sind, vorzugsweise für unsubstituiertes C₅-C₇-Cycloalkyl. In den Verbindungen der Formeln (III.1) und (III.2) steht R⁵ vorzugsweise für Wasserstoff oder C₁-C₄-Alkyl. Speziell steht R⁵ für C₁-C₂-Alkyl. Besonders bevorzugt ist Allylacetat.

Ebenfalls bevorzugt wird als Verbindung A) ein Ester der Formeln (III.1) oder (III.2) eingesetzt, der sich von dem als Edukt zur Carbonylierung eingesetzten Alkohol der Formel (II.1) oder (II.2) ableitet. Erfindungsgemäß besonders bevorzugte Ester der Formel (III.1) sind Ester aus einer linearen oder verzweigten einbasigen C₁-C₃-Alkancarbonsäure und eines unter 3-Methy-1-penten-3-ol, 1-Hepten-3-ol, 1-Vinylcyclohexanol, Linalool und Nerolidol ausgewählten Alkohols. Insbesondere bevorzugt sind Linaloylacetat und Nerolidylacetat, speziell 6*E*-Nerolidylacetat. Eine erfindungsgemäß besonders bevorzugte Verbindung der Formel (III.2) ist Farnesylacetat.

Sofern die Verbindungen der Formeln (III.1) und (III.2) ein oder mehrere Asymmetriezentren aufweisen, können auch Enantiomerengemische oder Diastereomerengemische eingesetzt werden.

In der Variante 1, bei der man einen Allylalkohol, ausgewählt unter Verbindungen der allgemeinen Formeln (II.1) und (II.2) zur Reaktion einsetzt, erfolgt die Carbonylierung erfindungsgemäß in Gegenwart einer bezogen auf den eingesetzten Allylalkohol unterstöchiometrischen Menge einer Verbindung A), d.h. die Stoffmenge an Verbindung A) ist kleiner als die Stoffmenge an Allylalkohol. Das molare Verhältnis von Verbindung A) zum aus Verbindungen der Formeln (II.1) und (II.2) ausgewählten Allylalkohol, liegt üblicherweise im Bereich von 0,001 zu 1 bis etwa 0,95 zu 1. Bevorzugt beträgt die Gesamtmenge der Verbindung A) höchstens 50 Mol-%, vorzugsweise höchstens 40 Mol-%, bevorzugt höchstens 30 Mol-%, bezogen auf die Gesamtmolmenge der Verbindungen (II.1) und (II.2). Molare Verhältnisse im Bereich von 0,1 zu 1 bis 0,35 zu 1 und speziell von 0,15 zu 1 bis 0,30 zu 1 sind besonders bevorzugt.

In den erfindungsgemäßen Verfahren zur Herstellung der Carbonsäure der Formel (I) ist die Wirkung des zugesetzten Anhydrids äquivalent zu der Wirkung des zugesetzten acylierten Allylalkohols der Formeln (III.1) und (III.2).

Weiterhin kann es zweckmäßig sein, die Carbonylierung nach Variante 1 in Gegenwart eines nucleophilen Reagenzes durchzuführen. Beispiele für nucleophile Reagentien sind 4-(C₁-C₄-Alkyl)pyridine, 4-(1-Pyrrolidinyl)pyridin und 4-(Di-(C₁-C₄-alkyl)amino)-pyridine. Geeignete 4-(C₁-C₄-Alkyl)pyridine sind 4-Methylpyridin und 4-Ethylpyridin. Geeignete 4-(Di-(C₁-C₄-alkyl)amino)pyridine sind 4-(Dimethylamino)pyridin und 4-(Diethylaminopyridin), insbesondere 4-Dimethylaminopyridin. Üblicherweise wird das nucleophile Reagenz ausgewählt aus 4-(C₁-C₄-Alkyl)pyridin, 4-(1-Pyrrolidinyl)pyridin und 4-(Di-(C₁-C₄-alkyl)amino)pyridin in einer Menge von 0,01 bis 5 Mol-%, bevorzugt von 0,05 bis 2 Mol-%, insbesondere von 0,1 bis 1 Mol-%, bezogen auf die Gesamtmolmenge der Verbindungen (11.1) und (II.2), eingesetzt.

Die folgenden Ausführungen zur Herstellung einer Zusammensetzung, enthaltend wenigstens eine ungesättigte Carbonsäure der allgemeinen Formel (I) beziehen sich auf alle Varianten des Verfahrens (die erfindungsgemäßen Varianten 1 und 3 und die nicht erfindungsgemäße Variante 2), sofern nicht ausdrücklich etwas anderes angegeben ist.

In allen Varianten wird die obere Grenze der Menge an Kohlenmonoxid durch ökonomische Gründe bestimmt, wobei die Verwendung einer überschüssigen Menge die Umsetzung nicht nachteilig beeinflusst. Vorteilhafterweise wird Kohlenmonoxid im Überschuss verwendet. Kohlenmonoxid wird gewöhnlicherweise in einer Menge von mindestens 1,1 Mol pro Gesamtmolmenge der Verbindungen (II.1) und (II.2) oder pro Gesamtmolmenge der Verbindungen (IV.1) und (IV.2) eingesetzt.

Die Carbonylierung (nach allen Varianten) erfolgt in Gegenwart eines Übergangsmetallkatalysators, der wenigstens ein Metall der Gruppen 8, 9 oder 10 des Periodensystems der Elemente umfasst. Als Übergangsmetalle werden bevorzugt Palladium, Ruthenium, Rhodium, Iridium und Eisen, besonders bevorzugt Palladium, eingesetzt.

Üblicherweise wird der Übergangsmetallkatalysator in einer Menge von höchstens 0,5 Mol-%, vor allem 0,001 bis 0,3 Mol-%, bezogen auf die Gesamtmenge der Verbindungen (II.1) und (II.2) oder bezogen auf die Gesamtmenge der Verbindungen (IV.1) und (IV.2), eingesetzt.

Erfindungsgemäß wird zusätzlich mindestens eine organische Phosphorverbindung als Ligand eingesetzt. Als organische Phosphorverbindungen eignen sich monodentate und bidentate Phosphorliganden. Auf die Art der organischen Phosphorverbindung kommt es prinzipiell nicht an, so dass sich im Allgemeinen die Verwendung von preiswerten tertiären monodentaten Phosphinen der Formel (V) 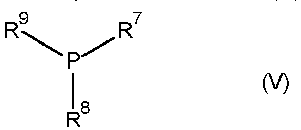 worin
- R⁷, R⁸ und R⁹: unabhängig voneinander ausgewählt sind aus unsubstituiertem und substituiertem Alkyl, unsubstituiertem und substituiertem Cycloalkyl, unsubstituiertem und substituiertem Heterocyclyl, unsubstituiertem und substituiertem Aryl und unsubstituiertes und substituiertes Hetaryl,
empfiehlt.

Vorzugsweise stehen R⁷, R⁸ und R⁹ unabhängig voneinander für
- C₁-C₁₈-Alkyl, das unsubstituiert ist oder mit Phenyl substituiert ist;
- C₅-C₈-Cycloalkyl, das unsubstituiert ist oder mit 1, 2 oder 3 C₁-C₆-Alkylresten substituiert ist;
- C₆-C₁₀-Aryl, das unsubstituiert ist oder mit 1, 2 oder 3 C₁-C₆-Alkylresten, C₁-C₆-Alkoxyresten, Di(C₁-C₄-dialkyl)amino, SO₃H oder SO₃M substituiert ist, wobei M für Li, Na, K, NH₄ steht;
- C₆-C₁₀-Aryl, das mit 1, 2 oder 3 Resten ausgewählt aus Fluor, Chlor und C₁-C₁₀-Fluoralkyl substituiert ist;
- 5- oder 6-gliedriges gesättigtes Heterocyclyl, enthaltend neben Kohlenstoffatomen ein Stickstoffatom oder ein Sauerstoffatom als Ringglied;
- C₅-C₈-Cycloalkyl; oder
- 5- oder 6-gliedriges Hetaryl, enthaltend neben Kohlenstoffatomen ein Heteroatom ausgewählt unter Stickstoff und Sauerstoff.

Beispiele für bevorzugte Phosphine der Formel (V) sind Trialkylphosphine, Triarylphosphine, Dialkylarylphosphine, Alkyldiarylphoshine, Cycloalkyldiarylphosphine, Dicycloalkylarylphosphine, und Tricycloalkylphosphine. Beispiele für bevorzugte Phosphine der Formel (V) sind ebenfalls Triheterocyclylphosphine und Trihetarylphosphine. Beispiele für bevorzugte Trialkylphosphine sind Triethylphosphin, Tri-n-butylphosphin, Tri-tert-butylphosphin, Tribenzylphosphin, etc. Beispiele für bevorzugte Tricycloalkylphosphine sind Tri(cyclopentyl)phosphin, Tri(cyclohexyl)phosphin, etc. Beispiele für bevorzugte Triarylphosphine sind Triphenylphosphin, Tri(p-tolyl)phosphin, Tri(m-tolyl)-phosphin, Tri (o-tolyl)phosphin, Tri(p-methoxyphenyl)phosphin, Tri(p-dimethylaminophenyl)phosphin, Tri-(natrium-meta-sulfonatophenyl)-phosphin, Diphenyl(2-sulfonatophenyl)phosphin, Tri(1-naphthyl)phosphin und Diphenyl-2-pyridylphosphin. Beispiele für bevorzugtes Dialkylarylphosphin sind Dimethylphenylphosphin und Di-tert-butylphenylphosphin. Beispiele für bevorzugte Alkyldiarylphoshine sind Ethyldiphenylphosphin und Isopropyldiphenylphosphin. Ein Beispiel für bevorzugtes Cycloalkyldiarylphosphin ist Cyclohexyldiphenylphosphin. Ein Beispiel für bevorzugtes Dicycloalkylarylphosphin ist Dicyclohexylphenylphosphin. Weitere Beispiele für bevorzugte Triarylphosphine sind Tri(o-methoxyphenyl)phosphin, Tri(m-methoxyphenyl)phosphin, Tri(p-fluorphenyl)phosphin, Tri(m-fluorphenyl)phosphin, Tri(o-fluorphenyl)phosphin, Tri(p-chlorphenyl)phosphin, Tri(m-chlorphenyl)phosphin, Tri(pentafluorphenyl)-phosphin, Tris(p-trifluormethylphenyl)phosphin, Tri[3,5-bis(trifluormethyl)phenyl]-phosphin, Diphenyl(o-methoxyphenyl)phosphin, Diphenyl(o-methylphenyl)phosphin, Tris(3,5-dimethylphenyl)phosphin und Tri-2-naphthylphosphin. Ein Beispiel für bevorzugtes Trihetarylphoshin ist Tri(o-furyl)phosphin. Ein weiteres Beispiel für bevorzugtes Trialkylphosphin ist Triisobutylphosphin. Ein Beispiel für bevorzugtes Trihetercyclylphosphin ist Tris(1-pyrrolidinyl)phosphin. Insbesondere bevorzugt sind Triphenylphosphin, Di-tert-butylphenylphosphin, Cyclohexyldiphenylphosphin, Dicyclohexylphenylphosphin Tri(p-tolyl)phosphin und Tri(cyclohexyl)phosphin.

Geeignete bidentate Phosphinliganden sind 2,2'-Bis(diphenyl-phosphino)-1,1'-binaphthyl (BINAP), 2-Bis(diphenylphosphino)ethan (DPPE), 1,3-Bis(diphenylphosphino)-propan (DPPP), 1,4-Bis(diphenylphosphino)butan (DPPB), 1,1'-Bis(diphenylphosphino)ferrocen (DPPF), 4,5-Bis(diphenylphosphino)-9,9-dimethylxanthen (Xantphos), 1,2-Bis(di-tert-butylphosphinomethyl)benzol, 1,2-Bis(di-tert-pentylphosphinomethyl)-benzol und 1,2-Bis(di-tert-butylphosphinomethyl)naphthalin.

Weiterhin kann es zweckmäßig sein, die Reaktion in Gegenwart von freiem, also nicht im Komplex gebundenem Phosphin der Formel (V) und/oder bidentaten Phosphinen durchzuführen.

Das Molverhältnis von organischer Phosphorverbindung zu Übergangsmetall beträgt üblicherweise 1:1 bis 10:1, vorzugsweise 2:1 bis 5:1. Speziell beträgt das Molverhältnis von organischer Phosphorverbindung der Formel (V) zu Übergangsmetall üblicherweise 1:1 bis 10:1, vorzugsweise 2:1 bis 5:1.

Es ist vorteilhaft, die Carbonylierung unter Ausschluss von oder verringertem Gehalt an Luftsauerstoff durchzuführen.

Zusätzlich zu den zuvor als Ligand beschriebenen organischen Phosphorverbindungen können die erfindungsgemäß eingesetzten Carbonylierungskatalysatoren noch wenigstens einen weiteren Liganden aufweisen, der vorzugsweise ausgewählt ist unter Halogeniden, Aminen, Acetat, Carboxylaten, Acetylacetonat, Aryl- oder Alkylsulfonaten, Hydrid, CO, Olefinen, Dienen, Cycloolefinen, Nitrilen, N-haltigen Heterocyclen, Aromaten und Heteroaromaten, Ethern sowie einzähnigen Phosphinit-, Phosphonit-, Phosphoramidit- und Phosphitliganden. Besonders geeignete weitere Liganden sind Carbonyl, Acetat, Acetylacetonat und Cycloolefine wie Cyclooctadien oder Norbornadien. Ebenfalls geeignet sind Halogenide, speziell Chloride und Bromide.

Geeignete Übergangsmetallquellen sind ganz allgemein Übergangsmetalle, Übergangsmetallverbindungen und Übergangsmetallkomplexe, woraus in der Reaktionszone unter den Carbonylierungsbedingungen der Carbonylsierungskatalysator in situ gebildet wird.

Geeignete Verbindungen der genannten Übergangsmetalle sind insbesondere solche, die im gewählten Reaktionsmedium löslich sind, wie beispielsweise Salze oder Komplexverbindungen mit geeigneten Liganden, wie z. B. Carbonyl, Acetylacetonat, Hydroxy, Cyclooctadien, Norbornadien, Cycloocten, Methoxy, Acetyl oder sonstige aliphatische oder aromatische Carboxylate. Im Rahmen des erfindungsgemäßen Verfahrens bevorzugte Übergangsmetallverbindungen sind Ru(II), Ru(III), Ru(IV)-, Ru(0)-, Rh(I)-, Rh(III)-, Rh(0)-, Ir(I)-, Ir(III), Ir(IV)-, Ir(0)-Verbindungen, Pd(II)-, Pd(IV)-, Pd(0)-, Pt(II)-, Pt(IV)- Pt(0)-Verbindungen sowie Fe(II)- und Fe(III)-Verbindungen.

Geeignete Rutheniumsalze sind beispielsweise Ruthenium(III)-chlorid, Ruthenium(IV)-, Ruthenium(VI)-oxid oder Ruthenium(VIII)-oxid, Alkalisalze der Rutheniumsauerstoffsäuren, wie K₂RuO₄ oder KRuO₄, oder Komplexverbindungen des Rutheniums. Dazu zählen die Metallcarbonyle, wie Trisrutheniumdodecacarbonyl oder Hexarutheniumoctadecacarbonyl, oder Mischformen, in denen CO teilweise durch Liganden der Formel PR⁷R⁸R⁹ ersetzt sind, wie Ru(CO)₃(P(C₆H₅)₃)₂ oder Tris(acetylacetonato)ruthenium(III).

Übergangsmetallquelle für geeignete Palladiumverbindungen oder -komplexe sind z. B. Palladium(II)-acetat, Palladium(II)-chlorid, Palladium(II)-bromid, Palladium(II)-nitrat, Palladium(II)-acetylacetonat, Palladium(0)-dibenzylidenaceton-Komplex, Palladium(0)-tetrakis(triphenylphosphin), Palladium(0)-bis(tri-o-tolylphosphin), Palladium(II)-propionat, Palladium(II)-bis(triphenylphosphin)dichlorid, Palladium(II)-nitrat, Palladium(II)-bis(acetonitril)dichlorid, Palladium(II)-bis(benzonitril)-dichlorid.

Als Übergangsmetallquelle geeignete Rhodiumverbindungen oder-komplexe sind z. B. Rhodium(II)- und Rhodium(III)-salze, wie Rhodium(II)- bzw. Rhodium(III)-carboxylat, Rhodium(II)- und Rhodium(III)-acetat, etc. Weiterhin eignen sich Rhodiumkomplexe, wie Rhodiumbiscarbonylacetylacetonat, Acetylacetonatobisethylenrhodium(I) Acetylacetonatocyclooctadienylrhodium(I), Acetylacetonatonorbornadienylrhodium(I), Acetylacetonatocarbonyltriphenylphosphinrhodium(I), Rh₄(CO)₁₂, Rh₆(CO)₁₆ etc.

Als Übergangsmetallquelle geeignete Eisenverbindungen sind Fe₂(CO)₁₀, Fe₂(CO)₉, Tris(acetylacetonato)eisen(III).

Der Katalysator kann homogen gelöst im flüssigen Reaktionsmedium oder bei einer mehrphasigen Umsetzung gelöst in der flüssigen Phase des Reaktionsmediums als sogenannter Homogenkatalysator oder geträgert oder ungeträgert als sogenannter Heterogenkatalysator eingesetzt werden.

Erfolgt die Umsetzung in Abwesenheit weiterer Reaktionspartner, so erhält man die Säure der Formel (I). Die erhaltene Säure der Formel (I) kann durch dem Fachmann in an sich bekannten Verfahren, wie z. B. durch Destillation, aus dem Reaktionsgemisch entfernt werden und der zurückbleibende Katalysator kann im Rahmen weiterer Umsetzungen genutzt werden. Die Säure der Formel (I) kann auch durch Extrahieren aus dem Reaktionsgemisch gewonnen.

Die Carbonylierung kann (nach allen Varianten) in Gegenwart oder Abwesenheit einer Base durchgeführt werden. Die Base ist von dem nucleophilen Reagenz verschieden. Geeignete Basen sind anorganische und organische Basen. Beispiele für bevorzugte anorganische Basen sind Alkalimetallcarbonate oder Erdalkimetallcarbonate. Bevorzugte Alkalimetalle sind Natrium und Kalium, besonders bevorzugt ist Natrium. Bevorzugte Erdalkimetalle sind Magnesium und Calcium. Geeignete Basen sind ebenfalls organische Basen, die von dem nucleophilen Reagenz, das ausgewählt ist aus 4-(C₁-C₄-Alkyl)pyridin, 4-(1-Pyrrolidinyl)pyridin und 4-(Di-(C₁-C₄-alkyl)amino)pyridin wie 4-(Dimethylamino)pyridin verschieden sind. Die Gegenwart der Base wie z. B. ein zusätzliches Amin fördert die Bildung des Salzes der Carbonsäure der allgemeinen Formel (I), so dass die Umsetzungsrate deutlich verbessert wird. Zudem werden Säuren, die sich unter den Reaktionsbedingungen aus den Anhydriden bilden, durch die Base neutralisiert oder gepuffert. Aus den Salzen der Carbonsäure der Formel (I) kann wiederum thermisch, beispielsweise durch Destillation des Reaktionsaustrages, oder durch Ansäuern die freie Säure generiert werden. Vorzugsweise erfolgt die Carbonylierung in Gegenwart eines von dem nucleophilen Reagenz wie 4-(Dimethylamino)pyridin verschiedenen Amins. Insbesondere wird ein tertiäres Amin oder ein basischer N-Heteroaromat eingesetzt.

Beispiele des tertiären Amins umfassen ein Trialkylamin, speziell ein Tri(C₁-C₁₅-Alkyl)-amin wie Trimethylamin, Triethylamin, Tripropylamin, Tributylamin, Trihexylamin, Trioctylamin, Tridecylamin, N,N-Dimethylethylamin, Dimethylpropylamin oder 1,4-Diazabicyclo[2.2.2]octan (DABCO); ein aromatisches Amin, wie Dimethylanilin oder Tribenzylamin; ein cyclisches Amin wie N-Methylpyrrolidin, N-Methylpiperidin, N-Methylmorpholin, N,N'-Dimethylpiperazin. Unter diesen ist ein Trialkylamin, wie Triethylamin bevorzugt. Ebenfalls geeignet sind N-Heteroaromaten wir Pyridin, N-Methylimidazol oder Chinolin. Die Base, die von dem nucleophilen Reagenz ausgewählt aus 4-(C₁-C₄-Alkyl)pyridin, 4-(1-Pyrrolidinyl)pyridin und 4-(Di-(C₁-C₄-alkyl)amino)pyridin wie 4-(Di-methylamino)pyridin verschieden ist, wird üblicherweise in einer Menge von 0,8 mol bis 1,2 mol pro Mol der Verbindung (I) eingesetzt.

Insbesondere bei Carbonylierung in Gegenwart eines Amins ist Ansäuern des Reaktionsgemisches und Extraktion ein geeignetes Verfahren, um die freie Säure zu erhalten. Alternativ kann die freie Säure destillativ aus dem Reaktionsgemisch abgetrennt werden, da das Salz der ungesättigten Säure der Formel (I) sich unter Einwirkung von Wärme in die freie ungesättigte Säure der Formel (I) und in das Amin zersetzt.

Die Carbonylierung (nach allen Varianten) kann in Gegenwart oder Abwesenheit eines zugesetzten inerten Lösungsmittels erfolgen. Als inerte Lösungsmittel sind Lösungsmittel zu verstehen, welche unter den Reaktionsbedingungen chemisch nicht mit den eingesetzten Verbindungen, d.h. den Edukten, den Produkten sowie den Katalysatoren, reagieren. Geeignete inerte Lösungsmittel sind aprotische, organische Lösungsmittel, beispielsweise aromatische Kohlenwasserstoffe wie Toluol oder Xylole, alizyklische Kohlenwasserstoffe wie Cyclohexan, und zyklische Ether wie Tetrahydrofuran oder Dioxan. Aus praktischen Gründen wird die Carbonylierung vorzugsweise in Abwesenheit eines inerten Lösungsmittels durchgeführt. Ist das eingesetzte Edukt jedoch unter den Reaktionsbedingungen schwerlöslich, ist die Verwendung eines Lösungsmittels ratsam.

Die Carbonylierung kann kontinuierlich, semikontinuierlich oder diskontinuierlich erfolgen.

Der Druck (nach allen Varianten) kann innerhalb eines breiten Bereichs wie von Atmosphärendruck bis höchstens 90 bar, vorzugsweise bis höchstens 30 bar, mehr vorzugsweise bis höchstens 25 bar, bevorzugt höchstens 20 bar und insbesondere höchstens 15 bar gewählt werden. Geeignete Druckbereiche sind beispielsweise 1,1 bis 90 bar, 1,1 bis 30 bar, 2 bis 20 bar, 5 bis 15 bar.

Die Reaktionstemperatur (nach allen Varianten) kann innerhalb eines breiten Bereichs von Raumtemperatur (25 °C) bis 100 °C gewählt werden, wobei sie jedoch vorzugsweise höchstens 80 °C, insbesondere höchstens 75 °C beträgt.

In einer speziellen Ausführungsform des erfindungsgemäßen Verfahrens erfolgt die Carbonylierung bei einer Temperatur von höchstens 90 °C und einem Druck von höchstens 20 bar.

Geeignete druckfeste Reaktoren sind dem Fachmann ebenfalls bekannt und werden z. B. in Ullmanns Enzyklopädie der technischen Chemie, Bd. 1, 3. Auflage, 1951, S. 769 ff. beschrieben. Im Allgemeinen wird für das erfindungsgemäße Verfahren ein Autoklav verwendet, der gewünschtenfalls mit einer Rührvorrichtung und einer Innenauskleidung versehen sein kann.

Die erfindungsgemäß verwendeten Katalysatoren lassen sich nach üblichen, dem Fachmann bekannten Verfahren vom Austrag der Carbonylierungsreaktion abtrennen und können erneut für eine Carbonylierung eingesetzt werden.

Das Reaktionsgemisch kann einer Aufarbeitung nach üblichen, dem Fachmann bekannten Verfahren unterzogen werden. Dazu kann das bei der Carbonylierung erhaltene Reaktionsgemisch wenigstens einem Aufarbeitungsschritt zur Abtrennung wenigstens einer der folgenden Komponenten:
- Carbonylierungskatalysator,
- nicht umgesetzten Verbindungen der Formel (II.1) und (II.2) bzw. (IV.1) und (IV.2),
- von den Verbindungen der Formel (I) verschiedenen Reaktionsprodukten,
- Lösungsmittel,
unterzogen werden.

Die Variante 2 betrifft, wie zuvor ausgeführt, die Carbonylierung eines unter Verbindungen der Formeln (IV.1) und (IV.2) ausgewählten acylierten Allylalkohols zur entsprechenden C1-verlängerten ungesättigten Carbonsäuren der Formel (I) oder ein Salz davon, umfassend die Umsetzung von unter Verbindungen der Formeln (IV.1) und (IV.2) ausgewählten Verbindungen mit Kohlenmonoxid in Gegenwart eines Übergangsmetallkatalysators, der wenigstens ein Metall der Gruppen 8, 9 oder 10 des Periodensystems der Elemente umfasst, wobei die Umsetzung zusätzlich in Gegenwart wenigstens einer organischen Phosphorverbindung als Ligand und in Gegenwart von Wasser erfolgt und wobei die Umsetzung bei einer Temperatur von höchstens 100 °C erfolgt.

Im Rahmen des nicht erfindungsgemäßen Verfahrens bevorzugte Verbindungen der Formeln (IV.1) und (IV.2) sind solche, worin die Reste R¹ und R² gleich oder verschieden sind und für Wasserstoff, lineares oder verzweigtes C₁-C₁₆-Alkyl, lineares oder verzweigtes C₂-C₁₆-Alkenyl mit einer oder zwei nicht konjugierten Doppelbindungen stehen oder R¹ und R² zusammen mit dem Kohlenstoffatom, an das sie gebunden sind, für unsubstituiertes C₅-C₇-Cycloalkyl oder C₅-C₇-Cycloalkyl, das 1, 2 oder 3 lineare oder verzweigte C₁-C₆-Alkylreste trägt, stehen.

Insbesondere bevorzugt werden Verbindungen der Formel (IV.1) und (IV.2) mit terpenartigen Kohlenwasserstoffresten. R¹ steht vorzugsweise für lineares oder verzweigtes C₆-C₁₆-Alkyl oder lineares oder verzweigtes C₆-C₁₆-Alkenyl mit einer oder zwei nicht konjugierten Doppelbindungen. R² steht vorzugsweise für Wasserstoff oder C₁-C₄-Alkyl, speziell C₁-C₂-Alkyl. R⁶ steht vorzugsweise für C₁-C₂-Alkyl. Besonders bevorzugt sind Ester aus einer linearen oder verzweigten einbasigen C₁-C₃-Alkancarbonsäure und eines unter 3-Methy-1-penten-3-ol, 1-Hepten-3-ol, 1-Vinylcyclohexanol, Linalool und Nerolidol ausgewählten allylischen Alkohols. Besonders bevorzugt sind Linaloylacetat und Nerolidylacetat, speziell 6*E*-Nerolidylacetat. Eine besonders bevorzugte Verbindung der Formel (IV.2) ist Farnesylacetat.

Sofern die Verbindungen der Formeln (IV.1) und (IV.2) ein oder mehrere Asymmetriezentren aufweisen, können auch Enantiomerengemische oder Diastereomerengemische eingesetzt werden. Das Verfahren kann mit einer Mischung der Verbindungen der Formeln (IV.1) und (IV.2) durchgeführt werden. Bevorzugt wird jedoch entweder eine Verbindung der Formel (IV.1) oder der Formel (IV.2) eingesetzt.

Verbindungen der Formeln (IV.1) sind beispielsweise durch Veresterung von Carbonsäuren der Formel R⁶-C(O)OH mit dem Allylalkohol der Formel (II.1) und Verbindungen der Formel (IV.2) sind beispielsweise durch Veresterung von Carbonsäuren der Formel R⁶-C(O)OH mit dem Allylalkohol der Formel (11.2) erhältlich.

Die Carbonylierung erfolgt in Gegenwart von Wasser. Das Molverhältnis von Wasser zur Gesamtmolmenge an Verbindungen der Formel (IV.1) und (IV.2) liegt üblicherweise im Bereich von 1 : 1 bis 10 : 1, bevorzugt jedoch in dem Bereich von 1,1: 1 bis 3 : 1.

Bezüglich geeigneter Übergangsmetallkatalysatoren, geeigneter organischen Phosphorverbindungen als Ligand, Reaktionstemperatur, Reaktionsdruck, Reaktionszeit, Verwendung von Lösungsmittel, Verwendung eines nucleophilen Reagenzes, Verwendung von dem nucleophilen Reagenz verschiedener Basen, speziell die Verwendung von Aminen, die von 4-(Dimethylamino)pyridin verschiedenen sind, und Reaktionsapparaturen wird auf das Zuvorgesagte Bezug genommen.

Eine weitere Ausführungsform der vorliegenden Erfindung (Variante 3) betrifft, wie zuvor ausgeführt, die Carbonylierung von Verbindungen der allgemeinen Formeln (II.1) und (II.2) zu den entsprechenden C-1 verlängerten ungesättigten Carbonsäuren der Formel (I) oder einem Salz davon, umfassend die Umsetzung von unter Verbindungen der Formeln (II.1) und (II.2) ausgewählten Verbindungen mit Kohlenmonoxid in Gegenwart eines Übergangsmetallkatalysators, der wenigstens ein Metall der Gruppen 8, 9 oder 10 des Periodensystems der Elemente umfasst, wobei die Umsetzung zusätzlich in Gegenwart wenigstens einer organischen Phosphorverbindung als Ligand und in Gegenwart einer bezogen auf den Allylalkohol unterstöchiometrischen Menge einer Verbindung B) und wobei die Umsetzung bei einer Temperatur von höchstens 100 °C erfolgt. In den Verbindungen B weisen R¹⁰ und R¹¹ vorzugsweise die gleiche Bedeutung auf. Als Verbindungen B) kommen vorzugsweise Methansulfonsäureanhydrid, Trifluormethansulfonsäureanhydrid, Phenylsulfonsäureanhydrid, p-Toluolsulfonsäureanhydrid, 4-Bromphenylsulfonsäureanhydrid und 4-Nitrophenylsulfonsäureanhydrid in Betracht.

In der Variante 3, bei der man einen Allylalkohol, ausgewählt unter Verbindungen der allgemeinen Formeln (II.1) und (II.2) zur Reaktion einsetzt, erfolgt die Carbonylierung erfindungsgemäß in Gegenwart einer bezogen auf den eingesetzten Allylalkohol unterstöchiometrischen Menge einer Verbindung B), d.h. die Stoffmenge an Verbindung B) ist kleiner als die Stoffmenge an Allylalkohol. Das molare Verhältnis von Verbindung B) zum aus Verbindungen der Formeln (II.1) und (II.2) ausgewählten Allylalkohol, liegt üblicherweise im Bereich von 0,001 zu 1 bis etwa 0,95 zu 1. Bevorzugt beträgt die Gesamtmenge der Verbindung B) höchstens 50 Mol-%, bevorzugt höchstens 40 Mol-%, bezogen auf die Gesamtmolmenge der Verbindungen (II.1) und (II.2). Molare Verhältnisse im Bereich von 0,1 zu 1 bis 0,35 zu 1 und speziell von 0,15 zu 1 bis 0,30 zu 1 sind besonders bevorzugt.

In den erfindungsgemäßen Verfahren zur Herstellung der Carbonsäure der Formel (I) nach Variante 3 ist die Wirkung des zugesetzten Sulfonsäureanhydrids äquivalent zu der Wirkung der zugesetzten Verbindung A) in der Variante 1.

Bezüglich geeigneter Übergangsmetallkatalysatoren, geeigneter organischen Phosphorverbindungen als Ligand, Reaktionstemperatur, Reaktionsdruck, Reaktionszeit, Verwendung von Lösungsmittel, Verwendung von nucleophilen Agentien, Verwendung von Basen, die von dem nucleophilen Reagenz verschieden sind, und Reaktionsapparaturen wird auf das Zuvorgesagte Bezug genommen.

Vorzugsweise erfolgt die Carbonylieung nach Variante 3 bei einer Temperatur von höchstens 100 °C und bei einem Druck von höchsten 30 bar.

Nach den Varianten 1 und 3 des erfindungsgemäßen Verfahrens gelingt es, Homofarnesylsäure, ausgehend von handelsüblichen Substanzen, wie Farnesol oder Nerolidol, unter milden Reaktionsbedingungen in hoher Ausbeute zu gewinnen.

Insbesondere eignet sich das erfindungsgemäße Verfahren zur Herstellung von E/Z-4,8-Dimethyl-3,7-nonadiensäure aus Linalool unter milden Reaktionsbedingungen.

Insbesondere eignet sich das erfindungsgemäße Verfahren ebenfalls zur Herstellung von (3E,7E)-4,8,12-Trimethyltrideca-3,7,11-triensäure oder ein Salz davon und (3Z,7E)-4,8,12-Trimethyltrideca-3,7,11-triensäure oder ein Salz davon in einem Gewichtsmengenverhältnis von 80 : 20 bis 50 : 50, bevorzugt von 70 : 30 bis 55 : 45, ausgehend von E-Nerolidol. Der Anteil an (3E,7E)-4,8,12-Trimethyltrideca-3,7,11-triensäure in den erhaltenen Isomerengemischen ist in der Regel höher als der Anteil an (3E,7E)-4,8,12-Trimethyltrideca-3,7,11-triensäure. Die erhaltene Zusammensetzung kann einer zumindest teilweisen Anreicherung eines Isomers unterzogen werden.

Bei den Verfahren nach den Varianten 1, 2 und 3 werden in der Regel E/Z-Isomerengemische der Formel (I) oder deren Salze erhalten, wenn R¹ und R² in Formel (I) unterschiedliche Bedeutungen aufweisen. Die E/Z-Isomerengemisch der Formel (I) enthalten eine 3-(E)-Säure der Formel (I-E) und eine 3-(Z)-Säure der Formel (I-Z), 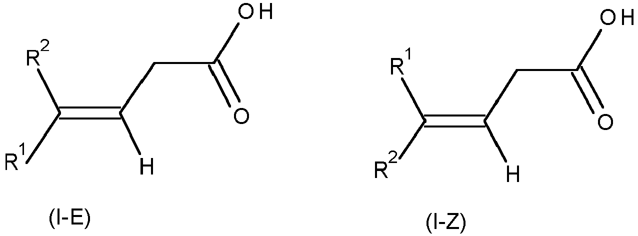 worin R¹ und R² unterschiedliche Bedeutungen aufweisen und R¹ nach IUPAC eine höhere Priorität aufweist.

Häufig ist nur eines der beiden Isomere das Wertprodukt. Vorzugsweise unterzieht man das nach dem erfindungsgemäßen Verfahren erhaltene E/Z-Isomerengemisch der Formel (I) einer zumindest teilweisen Anreicherung eines Isomers. Eine mögliche Isomerentrennung eines solchen Gemisches zur teilweisen Anreicherung eines Isomers kann chromatographisch, destillativ oder wie in EP 17157974.1 beschrieben erfolgen.

Häufig ist das Wertprodukt die 3-(E)-Säure der Formel (I-E) und nicht die ebenfalls in nennenswerten Mengen anfallende 3-(Z)-Säure der Formel (I-Z).

Es wäre daher wirtschaftlich sinnvoll, zunächst zumindest einen Teil der 3-(E)-Säure der Formel (I-E) aus der erfindungsgemäß erhaltenen Zusammensetzung, enthaltend das E/Z-Isomerengemisch der Formel I, abzutrennen und die nicht erwünschte 3-(Z)-Säure der Formel (I-Z) zumindest teilweise in das Wertprodukt, d. h. in die 3-(E)-Säure der Formel (I-E) umzuwandeln.

Daher umfasst in einer bevorzugten Ausführungsform der vorliegenden Erfindung das erfindungsgemäße Verfahren zur Herstellung eines E/Z-Isomerengemisches der Formel (I) die folgenden zusätzlichen Schritte:
(1) die das E/Z-Isomerengemisch der Formel (I) enthaltende Zusammensetzung wird in Gegenwart eines Alkohols und eines Lipase-Enzyms einer enzymkatalysierten Veresterung unterzogen, wobei die 3-(E)-Säure der Formel (I-E) zumindest teilweise zu einem 3-(E)-Ester umgesetzt wird, so dass eine Zusammensetzung erhalten wird, die den 3-(E)-Ester, nicht umgesetzte 3-(E)-Säure der Formel (I-E) und nicht umgesetzte 3-(Z)-Säure der Formel (I-Z), enthält;
(2) die in (1) erhaltene Zusammensetzung wird unter Erhalt einer Zusammensetzung, die an 3-(E)-Säure der Formel (I-E) abgereichert und an 3-(Z)-Säure der Formel (I-Z) angereichert ist, und unter Erhalt einer Zusammensetzung, die den 3-(E)-Ester enthält, aufgetrennt;
(3) die in (2) erhaltene Zusammensetzung, die an 3-(E)-Säure der Formel (I-E) abgereichert und an 3-(Z)-Säure der Formel (I-Z) angereichert ist, wird einer Isomerisierung zur Erhöhung des Gehalts an 3-(E)-Säure der Formel (I-E) unterzogen; und
(4) gegebenenfalls wird der in (2) erhaltene 3-(E)-Ester unter Erhalt der 3-(E)-Säure der Formel (I-E) gespalten.

Verfahren zu enzymatisch katalysierten Veresterungen eines Gemisches von 3-(E)-ungesättigter Carbonsäure und 3-(Z)-ungesättigter Carbonsäure mit einem Alkohol sind beispielsweise aus der EP 17157974.1 bekannt.

Viele Lipasen vermögen mit hoher Substratselektivität Veresterungen zu katalysieren. Bevorzugt handelt es sich bei der Lipase um Candida antarctica lipase (CALB) oder um eine auf einem polymeren Träger immobiliserte Form wie Novozym 435 ®. Die enzymkatalysierte Veresterung erfolgt vorzugsweise in Gegenwart eines aliphatischen Alkohols. Geeignete aliphatische Alkohole sind C₁-C₂₀-Alkanole wie Methanol, Ethanol, n-Propanol, iso-Propanol, n-Butanol, iso-Butanol, sec-Butanol, n-Pentanol, n-Hexanol, n-Heptanol und n-Octanol. Die enzymkatalysierte Veresterung kann gegebenenfalls in Gegenwart eines Verdünnungs- oder Lösungsmittels durchgeführt werden. Beispiele für geeignete Verdünnungs- oder Lösungsmittel sind insbesondere aliphatische Kohlenwasserstoffe wie Hexan, Cyclohexan, Heptan, Octan; aromatische Kohlenwasserstoffe wie z. B. Toluol, Xylen; Dialkylether wie Methyl-tert-butylether und Diisopropylether. Üblicherweise setzt man 1 - 5 Äquivalente Alkohol pro Äquivalent an 3-(E)-Säure ein. Die Veresterung wird üblicherweise bei einer Temperatur in einem Bereich von 0 bis 80 °C durchgeführt.

Die Lipase verestert die 3-(E)-Säure der Formel (I-E) mit Alkoholen sehr viel schneller als die entsprechende 3-(Z)-Säure der Formel (I-Z). Man erhält daher eine trennbare Zusammensetzung, die den 3-(E)-Ester, nicht umgesetzte 3-(E)-Säure der Formel (I-E) und nicht umgesetzte 3-(Z)-Säure der Formel (I-Z) enthält.

Die in Schritt (1) erhaltene Zusammensetzung kann durch Extraktion oder Destillation aufgetrennt werden.

In Schritt (4) wird gegebenenfalls der in Schritt (2) isolierte 3-(E)-Ester einer säure-, base- oder enzymkatalysierten Esterspaltung unterzogen, wobei die 3-(E)-Säure der Formel (I-E) oder deren Salz erhalten wird. Da die Verbindung der Formel (I-E) häufig das Wertprodukt ist, ist es zweckmäßig die Esterspaltung durchzuführen.

In einer weiteren bevorzugten Ausführungsform umfasst das erfindungsgemäße Verfahren zur Herstellung eines E/Z-Isomerengemisches der Formel (I) die folgenden zusätzlichen Schritte:
(i) die das E/Z-Isomerengemisch der Formel (I) enthaltende Zusammensetzung wird in Gegenwart eines Alkohols einer Veresterung unter Erhalt des 3-(E)-Ester und des 3-(Z)-Esters unterzogen;
(ii) die in (i) erhaltenen 3-(E)-Ester und des 3-(Z)-Ester werden einer Lipase katalysierten enzymatischen Verseifung unterzogen, wobei die Lipase den 3-(E)-Ester zumindeset teilweise zu der 3-(E)-Säure der Formel (I-E) spaltet, wobei eine Zusammensetzung erhalten wird, die die 3-(E)-Säure der Verbindung der Formel (I-E), nicht umgesetzten 3-(E)-Ester und nicht umgesetzten 3-(Z)-Ester enthält;
(iii) die in (ii) erhaltene Zusammensetzung wird aufgetrennt wird unter Erhalt einer Zusammensetzung, die die 3-(E)-Säure der Verbindung der Formel (I-E) enthält und unter Erhalt einer Zusammensetzung, die nicht umgesetzten 3-(E)-Ester und nicht umgesetzten 3-(Z)-Ester enthält;
(iv) die in (iii) erhaltene Zusammensetzung, die nicht umgesetzten 3-(E)-Ester und nicht umgesetzten 3-(Z)-Ester enthält, wird einer Esterspaltung unterzogen, wobei eine Zusammensetzung erhalten wird, die an 3-(E)-Säure der Formel (I-E) oder deren Salz abgereichert und an 3-(Z)-Säure der Formel (I-Z) oder deren Salz angereichert ist; und
(v) die in (iv) erhaltene Zusammensetzung, die an 3-(E)-Säure der Formel (I-E) abgereichert und an 3-(Z)-Säure der Formel (I-Z) angereichert ist, wird einer Isomerisierung zur Erhöhung des Gehalts an 3-(E)-Säure der Formel (I-E) unterzogen.

Bevorzugt erfolgt die Veresterung in Schritt (i) mit einem aliphatischen Alkohol. Geeignete aliphatische Alkohole sind C₁-C₂₀-Alkanole wie Methanol, Ethanol, n-Propanol, iso-Propanol, n-Butanol, iso-Butanol, sec-Butanol, n-Pentanol, n-Hexanol, n-Heptanol und n-Octanol. Die Veresterung kann säure-, basen- oder enzymkatalysiert durchgeführt werden. Die Veresterung wird üblicherweise bei einer Temperatur in einem Bereich von 0 bis 80 °C durchgeführt.

Verfahren zu enzymatisch katalysierten Esterspaltung eines Gemisches von 3-(E)-ungesättigten Carbonsäure und 3-(Z)-ungesättigten Carbonsäure in Gegenwart von Wasser sind beispielsweise aus der EP 17157974.1 bekannt.

Viele Lipasen vermögen mit hoher Substratselektivität Esterspaltungen zu katalysieren. In Schritt (ii) unterzieht man die in Schritt (i) erhaltene Zusammensetzung in Gegenwart von Wasser und eines Lipase-Enzyms einer enzymkatalysierten Esterspaltung, wobei man eine Zusammensetzung erhält, die 3-(E)-Säure der Formel (I-E), nicht umgesetzten 3-(E)-Ester und nicht umgesetzten 3-(Z)-Ester enthält. Bevorzugt handelt es sich bei der Lipase um Candida antarctica lipase (CALB) oder um eine auf einem polymeren Träger immobiliserte Form wie Novozym 435 ®.

Die in Schritt (ii) erhaltene Zusammensetzung kann durch Destillation oder Extraktion getrennt werden. Man erhält eine Zusammensetzung, die 3-(E)-Säure der Formel (I-E) enthält. Ebenfalls erhält man eine Zusammensetzung, die den 3-(E)-Ester und 3-(Z)-Ester enthält.

Die Esterspaltung in Schritt (iv) kann säure-, base- oder enzymkatalysiert erfolgen. Man erhält eine Zusammensetzung, die 3-(E)-Säure der Formel (I-E) oder deren Salz und 3-(Z)-Säure der Formel (I-Z) oder deren Salz enthält, wobei im Vergleich zu der in Schritt (i) eingesetzten Zusammensetzung der Gehalt an 3-(*E*)-Säure der Formel (I-E) erniedrigt und der Gehalt an 3-(*Z*)-Säure der Formel (I-Z) erhöht ist.

In den Schritten (3) bzw. (v) der zuvor genannten Verfahren wird die erhaltene Zusammensetzung einer Isomerisierung der 3-(Z)-Säure der Formel (I-Z) zu der 3-(E)-Säure der Formel (I-E) unterzogen, um den Anteil an Wertprodukt zu erhöhen. Die Isomerisierung erfolgt vorzugsweise in Gegenwart eines Anhydrids einer organischen Säure und einer Base. Geeignete Anhydride sind Anhydride aliphatischer C₁-C₁₂-Monocarbonsäuren, Anhydride aliphatischer C₄-C₂₀-Dicarbonsäuren, Anhydride cycloaliphatischer C₇-C₂₀-Dicarbonsäuren, Anhydride aromatischer C₃-C₂₀-Dicarbonsäuren, Anhydride aliphatischer Sulfonsäuren und Anhydride aromatischer Sulfonsäuren. Geeignete Basen sind Alkalimetallcarbonate, Erdalkalimetallcarbonate, Alkalimetallhydrogencarbonate, Erdalkalimetallhydrogencarbonate, Alkalimetallphosphate, Erdalkalimetallphosphate, Amine, basische N-Heteroaromaten, basische Ionenaustauscher und Mischungen davon. Die Isomerisierung erfolgt bevorzugt in Gegenwart eines Anhydrids einer organischen Säure und einer Base. Das Verfahren kann vorteilhafterweise bei einer Temperatur von 60 bis 175 °C, vorzugsweise 70 bis 160 °C, durchgeführt werden.

Der Druck ist nicht kritisch. Daher kann man die Isomerisierung bei Überdruck, Unterdruck oder bei Atmosphärendruck durchführen. Das Verfahren wird bei Verwendung einer Base und/oder eines Anhydrids mit einem Siedepunkt bei Normaldruck unterhalb der Reaktionstemperatur zweckmäßigerweise in einem Druckreaktor durchgeführt. Vorteilhafterweise kann die Isomerisierung auch unter den Bedingungen des erfindungsgemäßen Verfahrens durchgeführt werden. Vorteilhafterweise ist für die Isomerisierung die Anwesenheit eines Übergangmetallkatalysators nicht erforderlich.

Die nach dem erfindungsgemäßen Verfahren erhaltenen Verbindungen der allgemeinen Formel (I) können einer Reduktion unter Überführung der Säure- oder Estergruppe in die entsprechenden Alkohole unterzogen werden. In einer speziellen Ausführungsform wird zunächst ein Gemisch aus (3E,7E)-4,8,12-Trimethyltrideca-3,7,11-triensäure und (3Z,7E)-4,8,12-Trimethyltrideca-3,7,11-triensäure, erhältlich wie zuvor beschrieben, einer Trennung unter Erhalt von (3E,7E)-4,8,12-Trimethyltrideca-3,7,11-triensäure, beispielweise wie zuvor beschrieben, unterzogen und (3E,7E)-4,8,12-Trimethyltrideca-3,7,11-triensäure einer Reduktion unter Erhalt von (3E,7E)-Homofarnesol (VI) 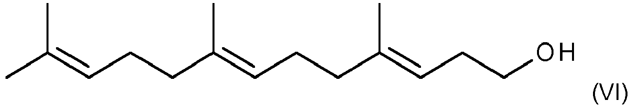 unterzogen. (3E,7E)-Homofarnesol (VI) ist ebenfalls ein wichtiges Zwischenprodukt zur Herstellung von Aromachemikalien.

Ein weiterer Gegenstand der Erfindung ist die Herstellung von (-)-Ambrox wie oben definiert. Die Herstellung kann nach bekannten Verfahren ausgehend von stereoisomerenreiner (3E/7E)-Homofarnesylsäure erfolgen, wie im folgenden Schema 1 gezeigt. 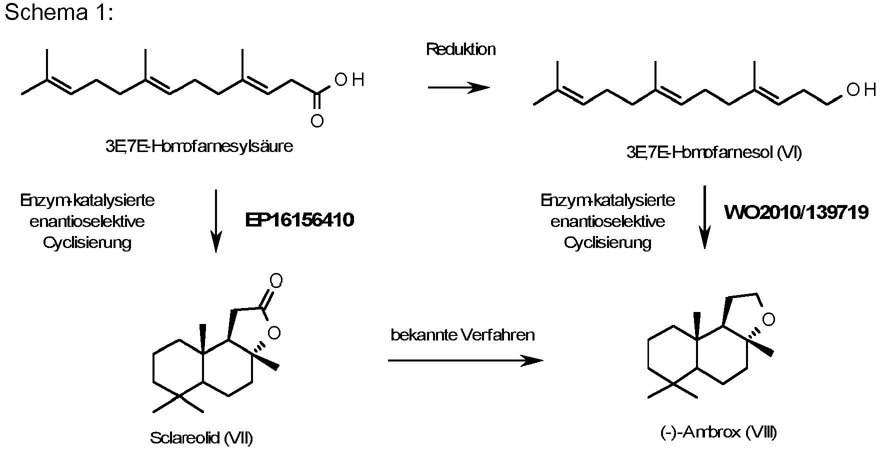 Die EP 16156410, WO 2010/139719 und WO 2012/066059 beschreiben die biokatalytische Zyklisierung von ungesättigten Substraten, z. B. von terpenartigen Kohlenwasserstoffen, unter Einsatz von Cyclase-Enzymen.

Sclareolid (VII) kann beispielsweise durch Cyclase-katalysierte Umwandlung von erfindungsgemäß hergestellter (3E/7E)-Homofarnesylsäure erhalten werden. Sclareolid (VII) kann dann chemisch, z. B. durch Umsetzung mit LiAlH₄ oder NaBH₄, unter Erhalt von Ambrox-1,4-diol reduziert werden (siehe Mookherjee et al.; Perfumer and Flavourist (1990), 15: 27). Ambrox-1,4-diol kann dann nach bekannten Verfahren weiter zu (-)-Ambrox umgesetzt werden.

Hydrierungskatalysatoren, die eine gute Selektivität bezüglich der Hydrierung der endständigen Carbonsäuregruppe unter Erhalt des entsprechenden Alkohols ermöglichen, sind dem Fachmann ebenfalls bekannt. Die Hydrierung und die Isomerisierung der in den Verbindungen (I) enthaltenen Doppelbindungen kann dabei im Wesentlichen vermieden werden.

Ebenso ist die Zyklisierung des (3*E*,7*E*)-Homofarnesols zu Ambrox bekannt, wobei sowohl enzymatische als auch chemische Cyclisierungen beschrieben sind. Diesbezüglich wird auf die folgenden Dokumente verwiesen.

P. F. Vlad et al. Khimiya Geterotsiklicheskikh Soedinenii, Engl. Transl. 1991, 746 beschreiben Cyclisierungsreaktionen unter Verwendung einer Supersäure (Fluorsulfonsäure in 2-Nitropropan). Ein weiteres geeignetes Verfahren umfasst die enantioselektive Polyencyclisierung von Homofarnesyltriethylsilylether in Gegenwart von O-(o-Fluorbenzyl)-binol und SnCl₄, wie von H. Yamamoto et al. J. Am. Chem. Soc. 2002, 3647 beschrieben.

Bevorzugt ist die biokatalytische Zyklisierung, wie sie in der WO 2010/139719 beschrieben ist. Danach erfolgt die Zyklisierung in Schritt d1) in Gegenwart eines Polypeptids mit der Aktivität einer Homofarnesol-Ambrox-Cyclase als Enzym.

Zur Herstellung von (-)-Ambrox nach dieser Variante wird Homofarnesol mit der Homofarnesol-Ambroxan-Cyclase in Kontakt gebracht und/oder inkubiert und anschließend das gebildete Ambrox isoliert.

In einer Ausführung wird Homofarnesol mit der Homofarnesol-Ambroxan-Cyclase in einem Medium in Kontakt gebracht und/oder so inkubiert, dass eine Umsetzung von Homofarnesol zu Ambrox in Gegenwart der Cyclase erfolgt. Bevorzugt handelt es sich bei dem Medium um ein wässriges Reaktionsmedium. Bei den wässrigen Reaktionsmedien handelt es sich vorzugsweise um gepufferte Lösungen, die in der Regel einen pH-Wert von vorzugsweise 5 bis 8 aufweisen. Als Puffer kann ein Citrat-, Phosphat-, TRIS- (Tris(hydroxymethyl)-aminomethan) oder MES-(2-(N-Morpholino)ethansulfonsäure)-Puffer verwendet werden. Ferner kann das Reaktionsmedium noch weitere Zusätze enthalten, wie z. B. Detergentien (beispielsweise Taurodeoxycholat).

Das Homofarnesol wird vorzugsweise in einer Konzentration von 5 bis 100 mM, besonders bevorzugt von 15 bis 25mM, in der enzymatischen Umsetzung eingesetzt. Diese kann kontinuierlich oder diskontinuierlich durchgeführt werden.

Die enzymatische Zyklisierung erfolgt zweckmäßig bei einer Reaktionstemperatur unterhalb der Desaktivierungstemperatur der eingesetzten Cyclase. Bevorzugt liegt die Reaktionstemperatur in Schritt d1) in einem Bereich von -10 bis 100 °C, besonders bevorzugt von 0 bis 80 °C, insbesondere von 15 bis 60 °C und speziell von 20 bis 40 °C.
Das Reaktionsprodukt Ambrox kann mit organischen Lösungsmitteln extrahiert werden und gegebenenfalls zur Aufreinigung destilliert werden. Geeignete Lösungsmittel werden im Folgenden genannt.

Neben einphasigen wässrigen Systemen können auch zweiphasige Systeme eingesetzt werden. In dieser Variante gelingt es, das gebildete Ambrox in der nicht-wässrigen Phase anzureichern, um es zu isolieren. Die zweite Phase ist dabei vorzugsweise ausgewählt unter ionischen Flüssigkeiten und organischen nicht-wasser-mischbaren Lösungsmitteln. Nach der Reaktion ist Ambrox in der organische Phase leicht von der wässrigen Phase, die den Biokatalysator enthält, abtrennbar. Unter nicht-wässrigen Reaktionsmedien werden Reaktionsmedien verstanden, die weniger als 1 Gew.-%, vorzugsweise weniger als 0,5 Gew.-% Wasser, bezogen auf das Gesamtgewicht des flüssigen Reaktionsmediums, enthalten. Insbesondere kann die Umsetzung in einem organischen Lösungsmittel durchgeführt werden. Geeignete organische Lösungsmittel sind beispielsweise aliphatische Kohlenwasserstoffe, vorzugsweise mit 5 bis 8 Kohlenstoffatomen, wie Pentan, Cyclopentan, Hexan, Cyclohexan, Heptan, Octan oder Cyclooctan, halogenierte aliphatische Kohlenwasserstoffe, vorzugsweise mit einem oder zwei Kohlenstoffatomen, wie Dichlormethan, Chloroform, Tetrachlorkohlenstoff, Dichlorethan oder Tetrachlorethan, aromatische Kohlenwasserstoffe, wie Benzol, Toluol, die Xylole, Chlorbenzol oder Dichlorbenzol, aliphatische acyclische und cyclische Ether oder Alkohole, vorzugsweise mit 4 bis 8 Kohlenstoffatomen, wie Ethanol, Isopropanol, Diethylether, Methyl-tert-butylether, Ethyl-tert-butylether, Dipropylether, Diisopropylether, Dibutylether, Tetrahydrofuran oder Ester wie Ethylacetat oder n-Butylacetat oder Ketone wie Methylisobutylketon oder Dioxan oder Gemische davon. Besonders bevorzugt werden die vorgenannten Heptan, Methyl-tert-butylether, Diisopropylether, Tetrahydrofuran, Ethylacetat verwendet.

Geeignete Enzyme sind in der WO 2010/139719 beschrieben. Danach ist das Enzym ein Polypeptid, welches kodiert wird von einem Nukleinsäuremolekül, umfassend mindestens ein Nukleinsäuremolekül, ausgewählt aus:
a) Nukleinsäuremolekül, das ein Polypeptid kodiert, umfassend die SEQ ID NO 2, 5, 6, 7, 8, 9, 10 oder 11; b) Nukleinsäuremolekül, das zumindest ein Polynukleotid der Sequenz gezeigt in SEQ ID NO 1 umfasst; c) Nukleinsäuremolekül, das ein Polypeptid kodiert, dessen Sequenz eine Identität von mindestens 46 % zu den Sequenzen SEQ ID NO 2, 5, 6, 7, 8, 9, 10 oder 11 aufweist; d) Nukleinsäuremolekül nach (a) bis (c), das für ein funktional äquivalentes Polypeptid oder ein Fragment der Sequenz gemäß SEQ ID NO 2, 5, 6, 7, 8, 9, 10 oder 11 steht; e) Nukleinsäuremolekül, kodierend für ein funktional äquivalentes Polypeptid mit der Aktivität einer Homofarnesol-Ambroxan-Cyclase, das man erhält, indem man ein Nukleinsäuremolekül aus einer cDNA Bank oder aus genomischer DNA mittels der Primer gemäß Sequenz Nr. 3 und 4 amplifiziert, oder das Nukleinsäuremolekül durch de novo-Synthese chemisch synthetisiert; f) Nukleinsäuremolekül, kodierend für ein funktional äquivalentes Polypeptid mit der Aktivität einer Homofarnesol-Ambroxan-Cyclase, das unter stringenten Bedingungen mit einem Nukleinsäuremolekül gemäß (a) bis (c) hybridisiert; g) Nukleinsäuremolekül, kodierend für ein funktional äquivalentes Polypeptid mit der Aktivität einer Homofarnesol-Ambroxan-Cyclase, das aus einer DNA-Bank unter Verwendung eines Nukleinsäuremoleküls gemäß (a) bis (c) oder deren Teilfragmente von mindestens 15 nt, vorzugsweise 20 nt, 30 nt, 50 nt, 100 nt, 200 nt oder 500 nt als Sonde unter stringenten Hybridisierungsbedingungen isoliert werden kann; und h) Nukleinsäuremolekül, kodierend für ein funktional äquivalentes Polypeptid mit der Aktivität einer Homofarnesol-Ambroxan-Cyclase, wobei die Sequenz des Polypeptids eine Identität von mindestens 46 % zu den Sequenzen SEQ ID NO 2, 5, 6, 7, 8, 9, 10 oder 1 1 aufweist; i) Nukleinsäuremolekül, kodierend für ein funktional äquivalentes Polypeptid mit der Aktivität einer Homofarnesol-Ambroxan-Cyclase, wobei das Polypeptid durch ein Nukleinsäuremolekül, ausgewählt aus der Gruppe der in a) bis h) beschriebenen, kodiert wird und mittels eines monoklonalen Antikörpers isoliert wurde oder isolierbar ist; j) Nukleinsäuremolekül, kodierend für ein funktional äquivalentes Polypeptid mit der Aktivität einer Homofarnesol-Ambroxan-Cyclase, wobei das Polypeptid eine analoge oder ähnliche Bindungsstelle aufweist, wie ein Polypeptid, kodiert durch ein Nukleinsäuremolekül, ausgewählt aus der Gruppe der in a) bis h) beschriebenen.

Trotz einer Vielzahl bereits vorhandener Aromachemikalien (Riech- und Geschmacksstoffe) und Verfahren zu ihrer Herstellung besteht ein ständiger Bedarf an neuen Komponenten, um die Vielzahl der für die äußerst diversen Einsatzbereiche gewünschten Eigenschaften erfüllen zu können sowie an einfachen Syntheserouten, um diese zugängig zu machen. Das erfindungsgemäße Verfahren ermöglicht die effektive Herstellung von Verbindungen der allgemeinen Formel (I) die als interessante Synthesebausteine bei der Bereitstellung von neuen und bereits bekannten Aromachemikalien, wie (-)-Ambrox, dienen können. Nach Hydrierung der Säurefunktion können Alkohole erhalten werden, die ihrerseits interessante Synthesebausteine sein können und sich für einen Einsatz als Tensidalkohole eignen.

Die Erfindung wird durch die nachstehenden Ausführungsbeispiele näher erläutert. Die nachfolgenden Beispiele belegen, dass die Erfindung vorteilhaft mit unterschiedlichen Edukten und Übergangsmetallkatalysatoren durchgeführt werden kann.

### Abkürzungen:

Ac₂O steht für Acetanhydrid (Essigsäureanhydrid); DMAP steht für 4-(Dimethylamino)-pyridin; eq. steht für Äquivalent; Fe(acac)₃ steht für Tris(acetylacetonato)eisen(III); Fl.-% steht für Flächenprozent; GC steht für Gaschromatographie; [Ir(COD)Cl]₂ steht für Bis(1,5-cyclooctadien)diiridium(I)-dichlorid; MTBE steht für Methyl-tert-butylether; NEt₃ steht für Triethylamin; org. steht für organisch; P(Cy)₃ steht für Tricyclohexylphosphin; Pd(acac)₂ steht für Bis(acetylacetonato)palladium(II); Pd(dba)₂ steht für Bis(dibenzylidenaceton)palladium(0); Pd(OAc)₂ steht für Palladium(II)-acetat; Rh(CO)₂(acac) steht für (Acetylacetonato)dicarbonylrhodium(I); Ru(acac)₃ steht für Tris(acetylacetonato)ruthenium(III); Sel. Säure steht für Selektivität Säure; THF steht für Tetrahydrofuran; TPP steht für Triphenylphosphin; U/pM steht für Umdrehungen pro Minute.

Die Produkte wurden mit Hilfe der GC-, ¹H-NMR und ¹³C-NMR-Analyse identifiziert. Die Aufarbeitung der Reaktionsgemische wurde nicht optimiert.

### Beispiel 1: Pd-katalysierte Carbonylierung ausgehend von Linalool

Palladium(II)-acetat (19 mg, 0,085 mmol), Triphenylphosphin (51 mg, 0,195 mmol) und DMAP (49 mg, 0,4 mmol) wurden unter Argon in einem Glasautoklaven vorgelegt. Linalool (10,8 g, 70 mmol), Triethylamin (6,9 g, 68 mmol) und Acetanhydrid (2 g, 20 mmol) wurden im Argongegenstrom zugegeben. Anschließend wurden 10 bar CO angelegt und bei diesem Druck wurde bei 60 °C Innentemperatur für 24 h bei 1000 U/pm gerührt. Danach wurde auf Raumtemperatur abgekühlt und entspannt. Eine GC-Analyse des Reaktionsaustrags ergab einen Umsatz von 94 % mit einer Selektivität zum Zielprodukt E/Z-4,8-Dimethyl-3,7-nonadiensäure von 62 %.

Nach beendeter Reaktion wurden MTBE (25 mL) und wässrige NaOH (5 %, 70 mL) zugegeben. Die Phasen wurden getrennt und die wässrige Phase wurde mit MTBE (2 x 50 mL) gewaschen. Die wässrige Phase wurde mit H₂SO₄ (20 %) auf pH = 1 gebracht und mit MTBE (3 x 50 mL) extrahiert. Die organischen Phasen aus der sauren Extraktion wurden vereinigt, mit Wasser neutral gewaschen (3 x 70 mL) und über MgSO₄ getrocknet. Nach dem Einengen erhielt man 7,1 g (56 %) der E/Z-4,8-Dimethyl-3,7-nonadiensäure mit einem E/Z-Verhältnis von 55:45 (¹H-NMR). Die NMR-Daten entsprechen den Daten von Snyder et al. Angew. Chem., 121, 2009, 8039. ¹³C-NMR (CDCl₃), E-Homomyrcenylcarbonsäure: 179.9, 139.5, 131.7, 125.1, 116.2, 40.2, 34.0, 27.2, 26.2, 18.0, 16.6; Z-Homomyrcenylcarbonsäure: 179.9, 139.5, 132.1, 124.8, 117.0, 34.0, 32.6, 27.0, 26.2, 23.7, 18.0.

### Beispiele 1.1 - 1.12: Pd-katalysierte Carbonylierung ausgehend von Linalool

Beispiel 1 wurde wiederholt. Die Einsatzstoffe zur Carbonylierung von Linalool, der Umsatz nach 24 h sowie die Selektivität zum Zielprodukt E/Z-4,8-Dimethyl-3,7-nonadiensäure sind in Tabelle I angegeben.

**Tabelle I: Carbonylierung von Linalool**

| Beispiel | Übergangsmetallverbindung / (Mol-%) | TPP (Mol-%) | DMAP (Mol-%) | Ac₂O (Mol-%) | CO (bar) | Umsatz nach 24h | Sel. Säure (FI.-% GC) |
|---|---|---|---|---|---|---|---|
| 1 | Pd(OAc)₂ / 0,12 | 0,28 | 0,6 | 28 | 10 | 94% | 62 |
| 1.1^{a)} | Pd(OAc)₂ / 0, 12 | 0,28 | 0,6 | 28 | 10 | 56% | 79 |
| 1.2^{b)} | Pd(OAc)₂ / 0,12 | 0,28 | 0,6 | 28 | 10 | 27% | 66 |
| 1.3^{c)} | Pd(OAc)₂ / 0,12 | 0,28 | 0,6 | 28 | 10 | 41% | 47 |
| 1.4^{d)} | Pd(OAc)₂ / 0,12 | 0,28 | 0,6 | 28 | 10 | 30% | 50 |
| 1.5 | Pd(acac)₂ / 0,12 | 0,28 | 0,6 | 28 | 10 | 80% | 70 |
| 1.6 | Pd(dba)₂ / 0,12 | 0,28 | 0,6 | 28 | 10 | 75% | 60 |
| 1.7^{e)} | PdCl₂ / 0,15 | 0,28 | 0,3 | 28 | 10 | 69% | 58 |
| 1.8 ^{f)} | Pd(OAc)₂ / 0,12 | 0,28 | 0,3 | 28 | 10 | 40% | 44 |
| 1.9^{e)} | Pd(OAc)₂ / 0,12 | 0,28 | 0,3 | 28 | 10 | 77% | 63 |
| 1.10^{g)} | PdCl₂ / 0,15 | 0,28 | - | - | 10 | 0% | - |
| 1.11^{f)} | PdCl₂ / 0,15 | 0,28 | - | - | 10 | 14% | 6 |
| 1.12^{f)} | Pd(OAc)₂/ 0,12 | 0,28 | - | - | 10 | 3% | - |

| | | | | | | | |
|---|---|---|---|---|---|---|---|
| a) Zusatz von 10 mL Pyridin anstatt NEt₃; b) Zusatz von 10 mL THF anstatt NEt₃; c) Zusatz von 10 mL Toluol anstatt NEt₃; d) Zusatz von 10 mL Dioxan anstatt NEt₃; e) 80 °C anstatt 60 °C; f) Zusatz von 10 mL THF anstatt NEt₃, 100 °C statt 60 °C; g) Zusatz von 10 mL THF anstatt NEt₃, 80 °C statt 60 °C. | | | | | | | |

Das Vergleichsbeispiel 1.10 zeigt, dass bei 10 bar bei unter den Bedingungen der EP 0146859 (PdCl₂/TPP) in Abwesenheit einer Anhydridquelle kein Umsatz erzielt wurde und auch bei 100 °C höchstens sehr geringe Umsätze zum Zielprodukt (Vergleichsbeispiel 1.11). Das Vergleichsbeispiel 1.12 zeigt, dass bei Verwendung von Pd(OAc)₂ in Abwesenheit einer Anhydridquelle nur ein minimaler Umsatz erzielt wurde.

### Beispiele 1.13 - 1.17: Carbonylierung ausgehend von Linalool unter Verwendung von Übergangsmetallkatalystoren der Gruppen 8 und 9 des Periodensystems

Beispiel 1 wurde wiederholt. Die Einsatzstoffe zur Carbonylierung von Linalool, der Umsatz nach 24 h sowie die Selektivität zum Zielprodukt (E/Z-4,8-Dimethyl-3,7-nonadiensäure) sind in Tabelle II angegeben

**Tabelle II: Carbonylierung von Linalool**

| Beispiel | Übergangsmetallverbindung | Übergangsmetall (Mol-%) | TPP/DMAP (Mol-%) | Ac₂O (Mol-%) | CO (bar) | Umsatz nach 24h | Sel. Säure (FI.%-GC) |
|---|---|---|---|---|---|---|---|
| 1.13 | Ru(acac)₃ | 0,12 | 0,28/0,3 | 28 | 10 | 56% | 60 |
| 1.14 | Ru₃(CO)₁₂ | 0,04 | 0,28/0,3 | 28 | 10 | 47% | 31 |
| 1.15 | Rh(CO)₂(acac) | 0,12 | 0,28/0,3 | 28 | 10 | 58% | 49 |
| 1.16 | Fe(acac)₂ | 0,12 | 0,28/0,3 | 28 | 10 | 36% | 49 |
| 1.17 | [Ir(COD)Cl]₂ | 0,06 | 0,28/0,3 | 28 | 10 | 49% | 27 |

### Beispiel 2: Carbonylierung von 3-Methyl-1-penten-3-ol

Palladium(II)-acetat (22 mg, 0,1 mmol), Triphenylphosphin (52 mg, 0,2 mmol) und DMAP (49 mg, 0,4 mmol) wurden unter Argon im Glasautoklaven vorgelegt. 3-Methyl-1-penten-3-ol (6,7 g, 67 mmol), Triethylamin (6,7 g, 67 mmol) und Acetanhydrid (2 g, 20 mmol) wurden im Argongegenstrom zugegeben. Anschließend wurden 10 bar CO angelegt und bei diesem Druck wurde bei 60 °C Innentemperatur für 24 h bei 1000 U/pm gerührt. Danach wurde auf Raumtemperatur abgekühlt und entspannt. Eine GC-Analyse des Reaktionsaustrags ergab einen Umsatz von 70 % mit einer Selektivität zum Zielprodukt E/Z-(4-Methyl)-3-hexensäure von 75 %.

Nach beendeter Reaktion wurden MTBE (25 mL) und wässrige NaOH (5 %, 50mL) zugegeben. Die Phasen wurden getrennt und die wässrige Phase wurde mit MTBE (2 x 25 mL) gewaschen. Die wässrige Phase wurde mit H₂SO₄ (20 %) auf pH = 1 gebracht und mit MTBE (3 x 25 mL) extrahiert. Die organischen Phasen aus der sauren Extraktion wurden vereinigt, mit Wasser neutral gewaschen (5 x 25 mL) und über MgSO₄ getrocknet. Nach dem Einengen erhielt man 2,3 g (27 %) E/Z-(4-Methyl)-3-hexensäure als leicht gelbes Öl mit einem E/Z-Verhältnis von 3:2 (¹H-NMR). ¹³C-NMR (d₈-Toluol): E-(4-Methyl)-3-hexensäure: 179.7, 140.8, 114.5, 33.7, 32.5, 16.1, 12.6; Z-(4-Methyl)-3-hexensäure: 179.7, 141.1, 115.5, 33.4, 25.2, 22.8, 12.6.

### Beispiel 3: Carbonylierung von 1-Hepten-3-ol

Palladium(II)-acetat (22 mg, 0,1 mmol), Triphenylphosphin (52 mg, 0,22 mmol) und DMAP (55 mg, 0,45 mmol) wurden unter Argon im Glasautoklaven vorgelegt. 1-Hepten-3-ol (7,6 g, 66 mmol), Triethylamin (6,7 g, 67 mmol) und Acetanhydrid (1,8 g, 17 mmol) wurden im Argongegenstrom zugegeben. Anschließend wurden 10 bar CO angelegt und bei diesem Druck wurde bei 60 °C Innentemperatur für 24 h bei 1000 U/pm gerührt. Danach wurde auf Raumtemperatur abgekühlt und entspannt. Eine GC-Analyse des Reaktionsaustrags ergab einen Umsatz von 75 % mit einer Selektivität bezüglich des Zielproduktes E/Z-3-Octensäure von 50 %.

Nach beendeter Reaktion wurden MTBE (25 mL) und wässrige NaOH (5 %, 50 mL) zugegeben. Die Phasen wurden getrennt und die wässrige Phase wurde mit MTBE (2 x 50 mL) gewaschen. Die wässrige Phase wurde mit H₂SO₄ (ca. 20 mL, 20 %) auf pH = 1 gebracht und mit MTBE (3 x 50 mL) extrahiert. Die organischen Phasen aus der sauren Extraktion wurden vereinigt, mit Wasser neutral gewaschen (4 x 50 mL) und über MgSO₄ getrocknet. Nach dem Einengen erhielt man 2,4 g (26 %) E/Z-3-Octensäure mit einem E/Z-Verhältnis von 78:22 (¹H-NMR). Die NMR-Daten entsprechen den Daten aus Wirth et al. Org. Lett., 9, 2007, 3169. ¹³C-NMR (d₈-Toluol), E-3-Octensäure: 179.4, 135.0, 121.4, 38.0, 32.5, 31.6, 22.6, 14.1.

### Beispiel 4: Carbonylierung von E-Nerolidol

Palladium(II)-acetat (46 mg, 0,2 mmol), Triphenylphosphin (120 mg, 0,46 mmol) und DMAP (56 mg, 0,46 mmol) wurden unter Argon im Stahlautoklaven vorgelegt. E-Nerolidol (34 g, 152,6 mmol), Triethylamin (17 g, 167 mmol) und Acetanhydrid (3,6 g, 35 mmol) wurden im Argongegenstrom zugegeben. Anschließend wurden 10 bar CO angelegt und bei diesem Druck wurde bei 70 °C Innentemperatur für 24 h bei 1000 U/pM gerührt. Nach 24 h wurde auf Raumtemperatur gekühlt und entspannt. Eine GC-Analyse des Reaktionsaustrags ergab einen Umsatz von 95 % mit einer Selektivität zum Zielprodukt E/Z-Homofarnesylsäure von 81 %.

Nach beendeter Reaktion wurde das Rohproduckt durch Kugelrohdestillation (1 mbar, bis 170 °C) aufgereinigt. Man erhielt 23 g (61 %) E/Z-Homofarnesylsäure mit einer Reinheit (GC) von 97 % mit einem Isomerenverhältnis von E-Homofarnesylsäure : Z-Homofarnesylsäure gleich 64 : 36 (GC).

¹³C-NMR (CDCl₃): E-Homofarnesylsäure: 179.1, 139.8, 135.4, 131.4, 124.4, 123.8, 114.9, 33.6, 39.8, 39.6, 26.8, 25.8, 26.4, 17.8, 16.5, 16.1; Z-Homofarnesylsäure: 179.2, 139.7, 135.7, 131.2, 124.5, 123.7, 115.9, 39.8, 33.5, 32.2, 26.8, 26.3, 25.7, 23.4, 17.7, 16.0.

Beispiel 4 wurde wiederholt. Die Einsatzstoffe zur Carbonylierung von E-Nerolidol, Umsatz nach 24 h und 6 h sowie die Ausbeute an Zielprodukt E/Z-Homofarnesylsäure (Summe beider Isomere) sind in Tabelle III angegeben.

**Tabelle III: Carbonylierung von E-Nerolidol**

| Beispiel | Pd(OAc)₂ (Mol-%) | TPP (Mol-%) | DMAP (Mol-%) | Ac₂O (Mol-%) | CO (bar) | Umsatz nach 24 h / (6 h) | Ausbeute^{#} (FI.-% GC) |
|---|---|---|---|---|---|---|---|
| 4 | 0,13 | 0,3 | 0,3 | 23 | 10 | 95% / (65%) | 77% |
| 4.1 | 0,13 | 0,3 | - | - | 10 | 0% | - |
| 4.2 | 0,13 | 1,3 | 0,3 | 23 | 10 | 50% | 33% |
| 4.3 | 0,13 | - | 0,3 | 23 | 10 | 26% | 1% |
| 4.4 | 0,13 | 0,3 | 0,3 | - | 10 | 0% | - |
| 4.5 | 0,13 | 0,3 | - | 23 | 10 | 94% / (54%) | 77% |
| 4.6^{a)} | 0,13 | 0,3 | 0,3 | 23 | 10 | 70% | 54% |
| 4.7 | 0,13 | 0,3 | 0,3 | b) | 10 | 52% | 32% |
| 4.8 | 0,13 | 0,3 | 0,3 | c) | 10 | >99% | 95% |
| 4.9 | 0,13 | 0,3 | - | d) | 10 | >99% | 58% |
| 4.10^{e)} | 0,13 | 0,3 | 0,3 | 23 | 10 | 34% | 16% |
| 4.11^{f)} | 0,13 | 0,3 | 0,3 | 23 | 10 | 30% | 18% |
| 4.12 | 0,13 | g) | 0,3 | 23 | 10 | 76% | 58% |
| 4.13 | 0,13 | h) | 0,3 | 23 | 10 | 83% | 72% |
| 4.14 | 0,13 | 0,3 | 0,3 | 23 | 20 | 99% | 85% |
| 4.15 | 0,13 | 0,3 | 0,3 | 23 | 40 | 97% | 85% |
| 4.16 | 0,13 | 0,3 | 0,3 | 23 | 80 | 94% | 79% |
| 4.17 | 0,13 | i) | 0,3 | 23 | 20 | 93% | 72% |
| 4.18 | 0,13 | k) | 0,3 | 23 | 20 | 95% | 80% |
| 4.19 | 0,13 | 0,3 | 0,3 | 16 | 20 | 93% | 79% |

| | | | | | | | |
|---|---|---|---|---|---|---|---|
| a) halbe Menge an NEt₃; b) 23 Mol-% Allylacetat (CAS 591-87-7) anstatt Ac₂O; c) 30 Mol-% E-Nerolidylacetat anstatt Ac₂O; d) 30 Mol-% E-Nerolidylacetat anstatt Ac₂O; e) 120 °C; f) THF anstelle von NEt₃; g) 0,3 Mol-% P(Cy)₃ anstatt TPP; h) 0,3 Mol-%Tri(p-Tolyl)phosphin anstatt TPP; i) 0,3 Mol-% Di(tert-Butyl)phenylphosphin anstatt TPP; k) 0,3 Mol-% Cyclohexyldiphenylphosphin anstatt TPP; # Ausbeute bestimmt über FI.-% GC als Produkt aus Selektivität und Umsatz | | | | | | | |

### Beispiel 5: Carbonylierung von E-Nerolidylacetat (nicht erfindungsgemäß)

Palladium(II)-acetat (50 mg, 0,22 mmol), Triphenylphosphin (130 mg, 0,5 mmol) und DMAP (70 mg, 0,57 mmol) wurden unter Argon im Stahlautoklaven vorgelegt. E-Nerolidylacetat (42,5 g, 142 mmol), Triethylamin (9 g, 89 mmol) und Wasser (2,8 g, 156 mmol) wurden im Argongegenstrom zugegeben. Anschließend wurden 10 bar CO angelegt und bei diesem Druck wurde bei 70 °C Innentemperatur für 24 h bei 1000 U/pM gerührt. Danach wurde auf Raumtemperatur abgekühlt und entspannt. Eine GC-Analyse des Reaktionsaustrags ergab einen Umsatz von 97 % mit einer Selektivität bezüglich E/Z-Homofarnesylsäure von 71 %. Verhältnis von E-Homofarnesylsäure : Z-Homofarnesylsäure = 64 : 36 (GC).

Beispiel 5 wurde wiederholt. Die Einsatzstoffe zur Carbonylierung von E-Nerolidylacetat, Umsatz nach 4 h und 24 h sowie die Ausbeute zum Zielprodukt E/Z-Homofarnesylsäure (Summe beider Isomere) sind in Tabelle IV angegeben.

**Tabelle IV: Carbonylierung von E-Nerolidlacetat**

| Beispiel | Pd(OAc)₂ (Mol-%) | TPP (Mol-%) | DMAP (Mol-%) | H₂O (eq.) | CO (bar) | Umsatz nach 24 h (4 h) | GC-Ausb. Säure (FI.-%) | Bemerkung |
|---|---|---|---|---|---|---|---|---|
| 5 | 0,15 | 0,4 | 0,4 | 1,1 | 10 | 97% (93%) | 74% | 70°C |
| 5.1 | 0,13 | 0,3 | 0,3 | 1,1 | 10 | >99% (65%) | 69% | 70°C, THF anstatt NEt₃ |
| 5.2 | 0,13 | 0,3 | 0,3 | - | 10 | >99% | <20% | 70°C |
| 5.3 | 0,13 | 0,3 | - | 1,1 | 10 | >99% | 86% | 70°C |
| 5.4 | 0,13 | 0,3 | - | 1,0 | 10 | 97% | 84% | 50°C |

### Beispiel 6: Carbonylierung von 1-Vinylcyclohexanol

Palladium(II)acetat (22 mg, 0,1 mmol), Triphenylphosphin (54 mg, 0,21 mmol) und DMAP (50 mg, 0,41 mmol) wurden unter Argon im Glasautoklaven vorgelegt. 1-Vinylcyclohexanol (8,35 g, 66 mmol), Triethylamin (6,7 g, 67 mmol) und Acetanhydrid (1,8 g, 17 mmol) wurden im Argongegenstrom zugegeben und es wurden 10 bar CO angelegt. Es wurde bei 60 °C Innentemperatur für 20 h bei 1000 U/pm gerührt, der Druck wurde bei 10 bar gehalten, nach 48 h wurde auf Raumtemperatur gekühlt und entspannt. GC-Umsatz: 54 %; Selektivität bezüglich 3-Cyclohexylidenpropansäure: 63 %.

Nach beendeter Reaktion wurde MTBE (25 mL) und wässrige NaOH (5 %, 50 mL) zugegeben. Die Phasen wurden getrennt und die wässrige Phase wurde mit MTBE (2 x 50 mL) gewaschen. Die wässrige Phase wurde mit H₂SO₄ (ca. 30 mL, 20 %) auf pH = 1 gebracht und mit MTBE (3 x 50 mL) extrahiert. Die org. Phasen aus der sauren Extraktion wurden vereinigt, mit Wasser neutral gewaschen (4 x 50 mL) und über MgSO₄ getrocknet. Einengen ergab 3,1 g (31 %) 3-Cyclohexylidenpropansäure. ¹³C-NMR (d₈-Toluol): 180.0, 143.8, 112.9, 37.5, 33.3, 29.4, 29.0, 28.1, 27.4.

### Beispiel 7: Isomerentrennung einer Zusammensetzung, enthaltend E/Z-Homofarnesylsäure und Isomerisierung zur Anreicherung der E-Homofarnesylsäure

### Isomerentrennung:

Das in Beispiel 4 erhaltene Gemisch wurde in n-Heptan(100 g) gelöst, mit Isopropanol (10 g) und Novozym 435 (0.5 g) versetzt und ca 17 h bei 60 °C gerührt. Das Enzym wurde dann abfiltriert. Bei einer Temperatur von 0 °C gab man Methanol und Wasser zu und stellte den pH-Wert des Reaktionsgemisches durch Zugabe von wässriger NaOH auf pH 12-13 bei einer Temperatur unterhalb 10 °C ein. Man trennte die Phasen und erhielt eine organische Phase mit (3*E*,7*E*)-Homofarnesylsäureisopropylester als Hauptkomponente und eine wässrige Phase mit einer Zusammensetzung, die 3Z,7E-Homofarnesylsäure : 3E,7E-Homofarnesylsäure : 2E,7E-Homofarnesylsäure : weitere Verbindungen im Verhältnis 68 % : 14 % : 17 % : 1 % enthielt (HPLC).

### Isomerisierung:

Palladium(II)-acetat (22 mg, 0,098 mmol), Triphenylphosphin (58 mg, 0,22 mmol) und DMAP (25 mg, 0,2 mmol) wurden unter Argon im Stahlautoklaven vorgelegt. Das Säuregemisch aus 3Z,7E-Homofarnesylsäure : 3E,7E-Homofarnesylsäure : 2E,7E-Homofarnesylsäure : weitere Verbindungen (im Verhältnis 68 % : 14 % : 17 % : 1 %) (17,5 g, 51 mmol), Triethylamin (8,1 g, 80 mmol) und Acetanhydrid (1,7 g, 17 mmol) wurden im Argongegenstrom zugegeben und es wurden 20 bar CO angelegt. Anschließend wurde bei 140 °C Innentemperatur für 14 h gerührt (1000 U/min). Der Druck wurde dabei bei 20 bar gehalten. Anschließend wurde auf Raumtemperatur gekühlt und entspannt. Man erhielt eine Zusammensetzung, die 3Z,7E-Homofarnesylsäure : 3E,7E-Homofarnesylsäure : 2E,7E-Homofarnesylsäure : weitere Verbindungen im Verhältnis 22 % : 33 % : 21 % : 24 % enthielt (HPLC).

## Patentansprüche

1. Verfahren zur Herstellung einer Zusammensetzung, enthaltend wenigstens eine ungesättigte Carbonsäure der allgemeinen Formel (I) 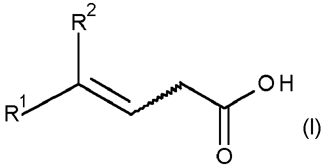 oder eines Salzes davon, worin
R¹ für Wasserstoff, lineares oder verzweigtes C₁-C₂₄-Alkyl, lineares oder verzweigtes C₂-C₂₄-Alkenyl mit 1, 2, 3 oder mehr als 3 C-C-Doppelbindungen, unsubstituiertes oder mit 1, 2 oder 3 C₁-C₆-Alkylresten substituiertes C₅-C₁₂-Cycloalkyl oder unsubstituiertes oder mit 1, 2 oder 3 C₁-C₆-Alkylresten substituiertes Aryl steht,
R² für Wasserstoff, lineares oder verzweigtes C₁-C₂₄-Alkyl oder lineares oder verzweigtes C₂-C₂₄-Alkenyl mit 1, 2, 3 oder mehr als 3 C-C-Doppelbindungen steht, oder
R¹ und R² gemeinsam mit dem Kohlenstoffatom, an das sie gebunden sind, für unsubstituiertes C₅-C₇-Cycloalkyl oder für C₅-C₇-Cycloalkyl, das 1, 2 oder 3 lineare oder verzweigte C₁-C₆-Alkylreste trägt, stehen,
bei dem man einen Allylalkohol, ausgewählt unter Verbindungen der allgemeinen Formeln (II.1) und (II.2) 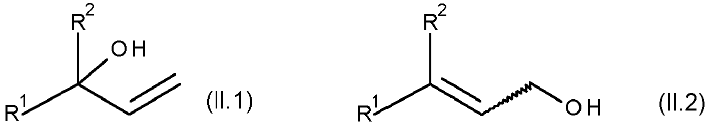 einer Carbonylierung durch Umsetzung mit Kohlenmonoxid in Gegenwart eines Übergangsmetallkatalysators unterzieht, der wenigstens ein Metall der Gruppen 8, 9 oder 10 des Periodensystems der Elemente umfasst, wobei die Umsetzung zusätzlich in Gegenwart wenigstens einer organischen Phosphorverbindung als Ligand und in Gegenwart einer bezogen auf den Allylalkohol unterstöchiometrischen Menge einer Verbindung A) erfolgt, die ausgewählt ist unter Anhydriden aliphatischer C₁-C₁₂-Monocarbonsäuren, Anhydriden aliphatischer C₄-C₂₀-Dicarbonsäuren, Anhydriden cycloaliphatischer C₇-C₂₀-Dicarbonsäuren, Anhydriden aromatischer C₈-C₂₀-Dicarbonsäuren und acylierten Allylalkoholen der Formeln (III.1) und (III.2) 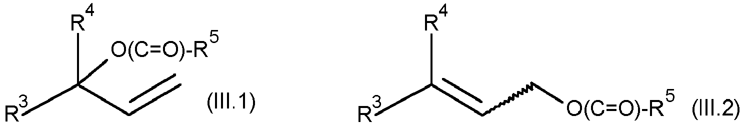 worin
R³ für Wasserstoff, lineares oder verzweigtes C₁-C₂₄-Alkyl, lineares oder verzweigtes C₂-C₂₄-Alkenyl mit 1, 2, 3 oder mehr als 3 C-C-Doppelbindungen, unsubstituiertes oder mit 1, 2 oder 3 C₁-C₆-Alkylresten substituiertes C₅-C₁₂-Cycloalkyl oder unsubstituiertes oder mit 1, 2 oder 3 C₁-C₆-Alkylresten substituiertes Aryl steht,
R⁴ für Wasserstoff, lineares oder verzweigtes C₁-C₂₄-Alkyl oder lineares oder verzweigtes C₂-C₂₄-Alkenyl mit 1, 2, 3 oder mehr als 3 C-C-Doppelbindungen steht, oder
R³ und R⁴ gemeinsam mit dem Kohlenstoffatom, an das sie gebunden sind, für unsubstituiertes C₅-C₇-Cycloalkyl oder für C₅-C₇-Cycloalkyl, das 1, 2 oder 3 lineare oder verzweigte C₁-C₆-Alkylreste trägt, stehen,
R⁵ für C₁-C₅-Alkyl steht,
und wobei die Umsetzung bei einer Temperatur von höchstens 100 °C erfolgt;
oder
bei dem man einen Allylalkohol, ausgewählt unter Verbindungen der allgemeinen Formeln (II.1) und (II.2) 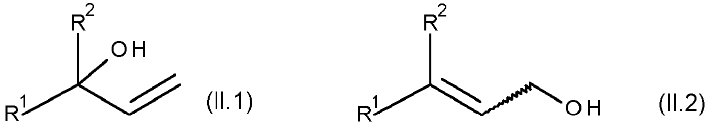 einer Carbonylierung durch Umsetzung mit Kohlenmonoxid in Gegenwart eines Übergangsmetallkatalysators unterzieht, der wenigstens ein Metall der Gruppen 8, 9 oder 10 des Periodensystems der Elemente umfasst, wobei die Umsetzung zusätzlich in Gegenwart wenigstens einer organischen Phosphorverbindung als Ligand und in Gegenwart einer bezogen auf den Allylalkohol unterstöchiometrischen Menge einer Verbindung B) 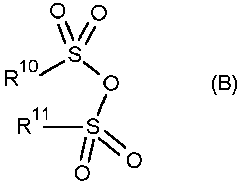 worin
R¹⁰ und R¹¹, unabhängig voneinander, für C₁-C₆-Alkyl, C₁-C₆-Fluoralkyl oder Phenyl, das unsubstituiert ist oder mit einem Substituenten ausgewählt aus Brom, Nitro und C₁-C₄-Alkyl substituiert ist, stehen;
und wobei die Umsetzung bei einer Temperatur von höchstens 100 °C erfolgt.

2. Verfahren nach Anspruch 1, wobei die Umsetzung bei einem Druck von höchstens 30 bar, vorzugsweise von höchstens 25 bar, bevorzugt von höchstens 20 bar, insbesondere von höchstens 15 bar erfolgt.

3. Verfahren nach einem der vorhergehenden Ansprüche, wobei die Carbonylierung nicht in Gegenwart einer zugesetzten Halogenwasserstoffsäure und nicht in Gegenwart eines zugesetzten Alkalimetall-, Erdalkalimetall- oder Ammoniumhalogenides erfolgt.

4. Verfahren nach einem der vorhergehenden Ansprüche, wobei der Halogenidgehalt des Reaktionsgemischs der Carbonylierung nicht mehr als 2 Mol-%, bevorzugt nicht mehr als 1 Mol-%, bezogen auf den Gesamtgehalt an Allylalkohol der allgemeinen Formeln (II.1) und (II.2) beträgt.

5. Verfahren nach einem der vorhergehenden Ansprüche, wobei die Verbindung A) ausgewählt ist unter Acetanhydrid und Allylacetat oder wobei als Verbindung A) ein Ester der Formel (III.1) oder (III.2) eingesetzt wird, der sich von dem als Edukt zur Carbonylierung eingesetzten Alkohol der Formel (II.1) oder (II.2) ableitet.

6. Verfahren nach einem der vorhergehenden Ansprüche, bei dem die Umsetzung zusätzlich in Gegenwart eines nucleophilen Reagenzes ausgewählt aus 4-(Di-(C₁-C₄-alkyl)amino)pyridin,4-(C₁-C₄-Alkyl)pyridin und 4-(1-Pyrrolidinyl)pyridin, bevorzugt von 4-(Dimethylamino)pyridin, erfolgt.

7. Verfahren nach einem der vorhergehenden Ansprüche, bei dem zur Umsetzung das Übergangsmetall in einer Menge von höchstens 0,5 Mol-%, bevorzugt von höchstens 0,3 Mol-%, bezogen auf die Gesamtmolmenge der Verbindungen (II.1) und (II.2), eingesetzt wird.

8. Verfahren nach einem der vorhergehenden Ansprüche, wobei das Übergangsmetall ausgewählt ist unter Pd, Ru, Rh, Ir und Fe, wobei bevorzugt als Übergangsmetall Pd eingesetzt wird.

9. Verfahren nach einem der vorhergehenden Ansprüche, wobei die organische Phosphorverbindung ausgewählt ist unter monodentaten und bidentaten Phosphinen, bevorzugt unter Trialkylphosphinen, Triarylphosphinen, Dialkylarylphosphinen, Alkyldiarylphosphinen, Cycloalkyldiarylphosphinen, Dicycloalkylarylphosphinen, Tricycloalkylphosphinen, Triheterocyclylphosphinen und Trihetarylphosphinen, wobei insbesondere als organische Phosphorverbindung Triphenylphosphin, Di-tert-butylphenylphosphin, Cyclohexyldiphenylphosphin, Dicyclohexylphenylphosphin, Tri(p-tolyl)phosphin oder Tricyclohexylphosphin eingesetzt wird.

10. Verfahren nach einem der vorhergehenden Ansprüche, bei dem die Gesamtmenge der Verbindung A) höchstens 50 Mol-%, bevorzugt höchstens 30 Mol-%, bezogen auf die Gesamtmolmenge der Verbindung (II.1) und (II.2), beträgt.

11. Verfahren nach einem der vorhergehenden Ansprüche, wobei die Umsetzung bei einer Temperatur von höchstens 80 °C, bevorzugt von höchstens 75 °C, erfolgt.

12. Verfahren nach einem der vorhergehenden Ansprüche, wobei zur Umsetzung eine Verbindung der allgemeinen Formel (II.1) eingesetzt wird, die ausgewählt ist unter Nerolidol, Linalool, 3-Methyl-1-penten-3-ol, 1-Hepten-3-ol und 1-Vinylcyclohexanol, speziell E-Nerolidol, oder wobei zur Umsetzung als Verbindung der allgemeinen Formel (II.2) Farnesol eingesetzt wird

13. Verfahren nach einem der vorhergehenden Ansprüche, wobei es sich bei der Zusammensetzung, die die wenigstens eine ungesättigte Carbonsäure der Formel (I) enthält, um ein E/Z-Isomerengemisch der Formel (I) handelt, enthaltend eine 3-(E)-Säure der Formel (I-E) und eine 3-(Z)-Säure der Formel (I-Z), 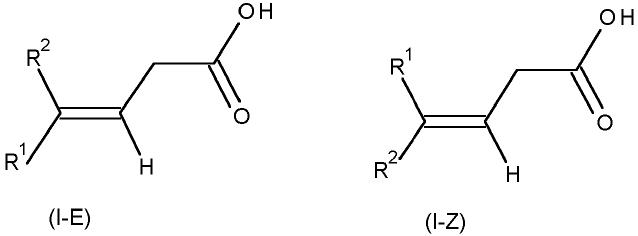 worin
R¹ und R² unterschiedliche Bedeutungen aufweisen und R¹ nach IUPAC eine höhere Priorität aufweist;
und man dieses E/Z-Isomerengemisch der Formel (I) einer Anreicherung eines Isomers unterzieht.

14. Verfahren nach Anspruch 13, wobei es sich bei der Zusammensetzung, die die wenigstens eine ungesättigte Carbonsäure der Formel (I) enthält, um ein E/Z-Isomerengemisch der Formel (I) handelt, enthaltend eine 3-(E)-Säure der Formel (I-E) und eine 3-(Z)-Säure der Formel (I-Z),
bei dem zusätzlich
(1) die das E/Z-Isomerengemisch der Formel (I) enthaltende Zusammensetzung in Gegenwart eines Alkohols und eines Lipase-Enzyms einer enzymkatalysierten Veresterung unterzogen wird, wobei die 3-(E)-Säure der Formel (I-E) zumindest teilweise zu einem 3-(E)-Ester umgesetzt wird, so dass eine Zusammensetzung erhalten wird, die den 3-(E)-Ester, nicht umgesetzte 3-(E)-Säure der Formel (I-E) und nicht umgesetzte 3-(Z)-Säure der Formel (I-Z), enthält;
(2) die in (1) erhaltene Zusammensetzung unter Erhalt einer Zusammensetzung, die an 3-(E)-Säure der Formel (I-E) abgereichert und an 3-(Z)-Säure der Formel (I-Z) angereichert ist, und unter Erhalt einer Zusammensetzung, die den 3-(E)-Ester enthält, aufgetrennt wird;
(3) die in (2) erhaltene Zusammensetzung, die an 3-(E)-Säure der Formel (I-E) abgereichert und an 3-(Z)-Säure der Formel (I-Z) angereichert ist, einer Isomerisierung zur Erhöhung des Gehalts an 3-(E)-Säure der Formel (I-E) unterzogen wird; und
(4) gegebenenfalls der in (2) erhaltene 3-(E)-Ester unter Erhalt der 3-(E)-Säure der Formel (I-E) gespalten wird;. oder
wobei es sich bei der Zusammensetzung, die die wenigstens eine ungesättigte Carbonsäure der Formel (I) enthält, um ein E/Z-Isomerengemisch der Formel (I) handelt, enthaltend eine 3-(E)-Säure der Formel (I-E) und eine 3-(Z)-Säure der Formel (I-Z), bei dem zusätzlich
(i) die das E/Z-Isomerengemisch der Formel (I) enthaltende Zusammensetzung in Gegenwart eines Alkohols einer Veresterung unter Erhalt des 3-(E)-Ester und des 3-(Z)-Esters unterzogen wird;
(ii) die in (i) erhaltenen 3-(E)-Ester und des 3-(Z)-Ester einer Lipase katalysierten enzymatischen Verseifung unterzogen werden, wobei die Lipase den 3-(E)-Ester zumindest teilweise zu der 3-(E)-Säure der Formel (I-E) spaltet, wobei eine Zusammensetzung erhalten wird, die die 3-(E)-Säure der Verbindung der Formel (I-E), nicht umgesetzten 3-(E)-Ester und nicht umgesetzten 3-(Z)-Ester enthält;
(iii) die in (ii) erhaltene Zusammensetzung aufgetrennt wird unter Erhalt einer Zusammensetzung, die die 3-(E)-Säure der Verbindung der Formel (I-E) enthält und unter Erhalt einer Zusammensetzung, die nicht umgesetzten 3-(E)-Ester und nicht umgesetzten 3-(Z)-Ester enthält;
(iv) die in (iii) erhaltene Zusammensetzung, die nicht umgesetzten 3-(E)-Ester und nicht umgesetzten 3-(Z)-Ester enthält, einer Esterspaltung unterzogen wird, wobei eine Zusammensetzung erhalten wird, die an 3-(E)-Säure der Formel (I-E) oder deren Salz abgereichert und an 3-(Z)-Säure der Formel (I-Z) oder deren Salz angereichert ist; und
(v) die in (iv) erhaltene Zusammensetzung, die an 3-(E)-Säure der Formel (I-E) abgereichert und an 3-(Z)-Säure der Formel (I-Z) angereichert ist, einer Isomerisierung zur Erhöhung des Gehalts an 3-(E)-Säure der Formel (I-E) unterzogen wird.

15. Verfahren zur Herstellung von (-)-Ambrox (VIII) 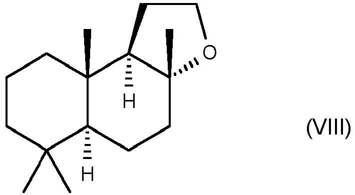 bei dem man
a1) nach einem Verfahren wie in einem der Ansprüche 1 bis 14 definiert ein Gemisch aus (3E,7E)-4,8,12-Trimethyltrideca-3,7,11-triensäure und (3Z,7E)-4,8,12-Trimethyltrideca-3,7,11-triensäure bereitstellt;
b1) das Gemisch aus (3E,7E)-4,8,12-Trimethyltrideca-3,7,11-triensäure und (3Z,7E)-4,8,12-Trimethyltrideca-3,7,11-triensäure einer Trennung unter Erhalt von (3E,7E)-4,8,12-Trimethyltrideca-3,7,11-triensäure unterzieht;
c1) die (3E,7E)-4,8,12-Trimethyltrideca-3,7,11-triensäure einer Reduktion unter Erhalt von (3E,7E)-Homofarnesol (VI) unterzieht;
d1) das (3E,7E)-Homofarnesol (VI) einer Zyklisierung unter Erhalt von (-)-Ambrox (VIII) unterzieht;
oder
a2) nach einem Verfahren wie in einem der Ansprüche 1 bis 14 definiert ein Gemisch aus (3E,7E)-4,8,12-Trimethyltrideca-3,7,11-triensäure und (3Z,7E)-4,8,12-Trimethyltrideca-3,7,11-triensäure bereitstellt;
b2) das Gemisch aus (3E,7E)-4,8,12-Trimethyltrideca-3,7,11-triensäure und (3Z,7E)-4,8,12-Trimethyltrideca-3,7,11-triensäure einer Trennung unter Erhalt von (3E,7E)-4,8,12-Trimethyltrideca-3,7,11-triensäure unterzieht;
c2) die (3E,7E)-4,8,12-Trimethyltrideca-3,7,11-triensäure einer Zyklisierung unter Erhalt von Sclareolid (VII) unterzieht;
d2) das Sclareolid (VII) einer Reduktion unter Erhalt von (-)-Ambrox (VIII) unterzieht.

## Claims

1. A process for preparing a composition comprising at least one unsaturated carboxylic acid of the general formula (I) 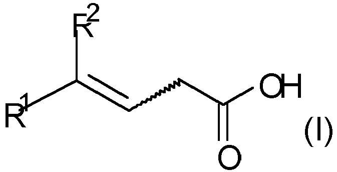 or a salt thereof, in which
R¹ is hydrogen, linear or branched C₁-C₂₄-alkyl, linear or branched C₂-C₂₄-alkenyl having 1, 2, 3 or more than 3 C-C double bonds, unsubstituted C₅-C₁₂cycloalkyl or C₅-C₁₂-cycloalkyl substituted by 1, 2 or 3 C₁-C₆-alkyl radicals or unsubstituted aryl or aryl substituted by 1, 2 or 3 C₁-C₆-alkyl radicals,
R² is hydrogen, linear or branched C₁-C₂₄-alkyl or linear or branched C₂-C₂₄-alkenyl having 1, 2, 3 or more than 3 C-C double bonds, or
R¹ and R² together with the carbon atom to which they are bonded are unsubstituted C₅-C₇-cycloalkyl or are C₅-C₇-cycloalkyl bearing 1, 2 or 3 linear or branched C₁-C₆-alkyl radicals,
in which an allyl alcohol selected from compounds of the general formulae (II.1) and (II.2) 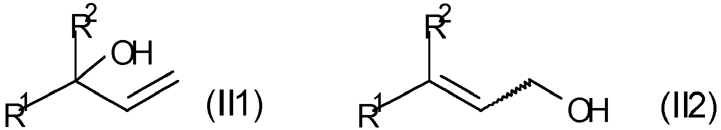
is subjected to a carbonylation by reaction with carbon monoxide in the presence of a transition metal catalyst comprising at least one metal of groups 8, 9 and 10 of the Periodic Table of the Elements, wherein the reaction is additionally effected in the presence of at least one organic phosphorus compound as ligand and in the presence of a substoichiometric amount, based on the allyl alcohol, of a compound A) selected from anhydrides of aliphatic C₁-C₁₂-monocarboxylic acids, anhydrides of aliphatic C₄-C₂₀-dicarboxylic acids, anhydrides of cycloaliphatic C₇-C₂₀-dicarboxylic acids, anhydrides of aromatic C₈-C₂₀-dicarboxylic acids and acylated allyl alcohols of the formulae (III.1) and (III.2) 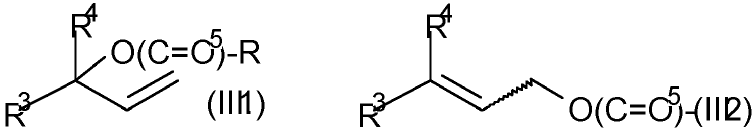 in which
R³ is hydrogen, linear or branched C₁-C₂₄-alkyl, linear or branched C₂-C₂₄-alkenyl having 1, 2, 3 or more than 3 C-C double bonds, unsubstituted C₅-C₁₂-cycloalkyl or C₅-C₁₂-cycloalkyl substituted by 1, 2 or 3 C₁-C₆-alkyl radicals or unsubstituted aryl or aryl substituted by 1, 2 or 3 C₁-C₆-alkyl radicals,
R⁴ is hydrogen, linear or branched C₁-C₂₄-alkyl or linear or branched C₂-C₂₄-alkenyl having 1, 2, 3 or more than 3 C-C double bonds, or
R³ and R⁴ together with the carbon atom to which they are bonded are unsubstituted C₅-C₇-cycloalkyl or are C₅-C₇-cycloalkyl bearing 1, 2 or 3 linear or branched C₁-C₆-alkyl radicals,
R⁵ is C₁-C₅-alkyl,
and wherein the reaction is effected at a temperature of not more than 100°C;
or
in which an allyl alcohol selected from compounds of the general formulae (II.1) and (II.2) 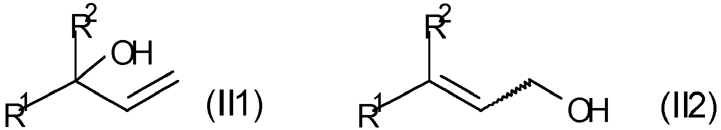 is subjected to a carbonylation by reaction with carbon monoxide in the presence of a transition metal catalyst comprising at least one metal of groups 8, 9 and 10 of the Periodic Table of the Elements, wherein the reaction is additionally effected in the presence of at least one organic phosphorus compound as ligand and in the presence of a substoichiometric amount, based on the allyl alcohol, of a compound B) 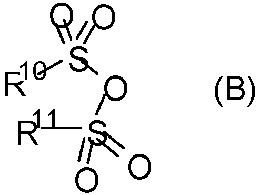 in which
R¹⁰ and R¹¹, independently of one another, are C₁-C₆-alkyl, C₁-C₆-fluoroalkyl or phenyl which is unsubstituted or substituted by a substituent selected from bromo, nitro and C₁-C₄-alkyl;
and wherein the reaction is effected at a temperature of not more than 100°C.

2. The process according to claim 1, wherein the reaction is effected at a pressure of not more than 30 bar, preferably of not more than 25 bar, more preferably of not more than 20 bar, especially of not more than 15 bar.

3. The process according to either of the preceding claims, wherein the carbonylation is not effected in the presence of an added hydrohalic acid and not in the presence of an added alkali metal halide, alkaline earth metal halide or ammonium halide.

4. The process according to any one of the preceding claims, wherein the halide content of the reaction mixture of the carbonylation is not more than 2 mol%, preferably not more than 1 mol%, based on the total content of allyl alcohol of the general formulae (II.1) and (II.2).

5. The process according to any one of the preceding claims, wherein the compound A) used is selected from acetic anhydride and allyl acetate, or wherein the compound A) used is an ester of the formula (III.1) or (III.2) that derives from the alcohol of the formula (II.1) or (II.2) used as the reactant for carbonylation.

6. The process according to any one of the preceding claims, in which the reaction is additionally effected in the presence of a nucleophilic reagent selected from 4-(di(C₁-C₄-alkyl)amino)pyridine, 4-(C₁-C₄-alkyl)pyridine and 4-(1-pyrrolidinyl)pyridine, preferably of 4-(dimethylamino)pyridine.

7. The process according to any one of the preceding claims, in which the transition metal is used for the reaction in an amount of not more than 0.5 mol%, preferably of not more than 0.3 mol%, based on the total molar amount of the compounds (II.1) and (II.2).

8. The process according to any one of the preceding claims, wherein the transition metal is selected from Pd, Ru, Rh, Ir and Fe, preference being given to using Pd as the transition metal.

9. The process according to any one of the preceding claims, wherein the organic phosphorus compound is selected from monodentate and bidentate phosphines, preferably from trialkylphosphines, triarylphosphines, dialkylarylphosphines, alkyldiarylphosphines, cycloalkyldiarylphosphines, dicycloalkylarylphosphines, tricycloalkylphosphines, triheterocyclylphosphines and trihetarylphosphines, the organic phosphorus compound used especially being triphenylphosphine, di-tert-butylphenylphosphine, cyclohexyldiphenylphosphine, dicyclohexylphenyl-phosphine, tri(p-tolyl)phosphine or tricyclohexylphosphine.

10. The process according to any one of the preceding claims, in which the total amount of the compound A) is not more than 50 mol%, preferably not more than 30 mol%, based on the total molar amount of the compound (II.1) and (II.2).

11. The process according to any one of the preceding claims, wherein the reaction is effected at a temperature of not more than 80°C, preferably of not more than 75°C.

12. The process according to any one of the preceding claims, wherein a compound of the general formula (II.1) which is used for the reaction is selected from nerolidol, linalool, 3-methyl-1-penten-3-ol, 1-hepten-3-ol and 1-vinylcyclohexanol, particularly E-nerolidol, or wherein the compound of the general formula (II.2) used for the reaction is farnesol.

13. The process according to any one of the preceding claims, wherein the composition comprising the at least one unsaturated carboxylic acid of the formula (I) is an E/Z isomer mixture of the formula (I) comprising a 3-(E) acid of the formula (I-E) and a 3-(Z) acid of the formula (I-Z) 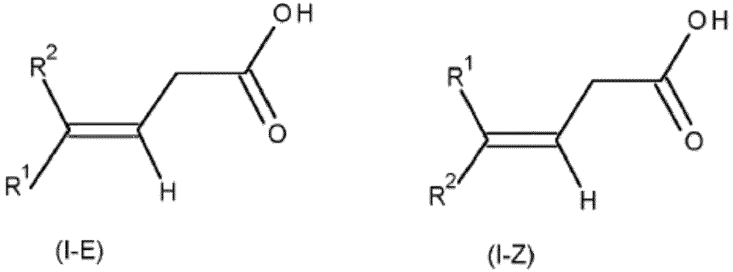 in which
R¹ and R² have different definitions and R¹ has a higher priority according to IUPAC;
and this E/Z isomer mixture of the formula (I) is subjected to an enrichment of one isomer.

14. The process according to claim 13, wherein the composition comprising the at least one unsaturated carboxylic acid of the formula (I) is an E/Z isomer mixture of the formula (I) comprising a 3-(E) acid of the formula (I-E) and a 3-(Z) acid of the formula (I-Z), in which, in addition,
(1) the composition comprising the E/Z isomer mixture of the formula (I), in the presence of an alcohol and of a lipase enzyme, is subjected to an enzyme-catalyzed esterification, wherein the 3-(E) acid of the formula (I-E) is converted at least partly to a 3-(E) ester, so as to obtain a composition comprising the 3-(E) ester, unconverted 3-(E) acid of the formula (I-E) and unconverted 3-(Z) acid of the formula (I-Z);
(2) the composition obtained in (1) is separated to obtain a composition depleted of 3-(E) acid of the formula (I-E) and enriched in 3-(Z) acid of the formula (I-Z), and to obtain a composition comprising the 3-(E) ester;
(3) the composition obtained in (2) which is depleted of 3-(E) acid of the formula (I-E) and enriched in 3-(Z) acid of the formula (I-Z) is subjected to an isomerization to increase the content of 3-(E) acid of the formula (I-E); and
(4) optionally, the 3-(E) ester obtained in (2) is cleaved to obtain the 3-(E) acid of the formula (I-E); or
wherein the composition comprising the at least one unsaturated carboxylic acid of the formula (I) is an E/Z isomer mixture of the formula (I) comprising a 3-(E) acid of the formula (I-E) and a 3-(Z) acid of the formula (I-Z), in which, in addition,
(i) the composition comprising the E/Z isomer mixture of the formula (I) is subjected to an esterification in the presence of an alcohol to obtain the 3-(E) ester and the 3-(Z) ester;
(ii) the 3-(E) ester and the 3-(Z) ester obtained in (i) are subjected to a lipase-catalyzed enzymatic hydrolysis, wherein the lipase at least partly cleaves the 3-(E) ester to give the 3-(E) acid of the formula (I-E) to obtain a composition comprising the 3-(E) acid of the compound of the formula (I-E), unconverted 3-(E) ester and unconverted 3-(Z) ester;
(iii) the composition obtained in (ii) is separated to obtain a composition comprising the 3-(E) acid of the compound of the formula (I-E) and to obtain a composition comprising unconverted 3-(E) ester and unconverted 3-(Z) ester;
(iv) the composition which is obtained in (iii) and comprises unconverted 3-(E) ester and unconverted 3-(Z) ester is subjected to an ester cleavage to obtain a composition depleted of 3-(E) acid of the formula (I-E) or salt thereof and enriched in 3-(Z) acid of the formula (I-Z) or salt thereof; and
(v) the composition which is obtained in (iv) and is depleted of 3-(E) acid of the formula (I-E) and enriched in 3-(Z) acid of the formula (I-Z) is subjected to an isomerization to increase the content of 3-(E) acid of the formula (I-E).

15. A process for preparing (-)-ambrox (VIII) 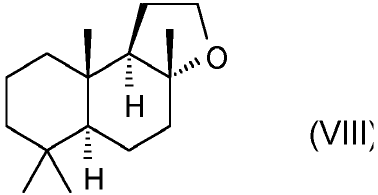 in which
a1) by a process as defined in any one of claims 1 to 14, a mixture of (3E,7E)-4,8,12-trimethyltrideca-3,7,11-trienoic acid and (3Z,7E)-4,8,12-trimethyltrideca-3,7,11-trienoic acid is provided;
b1) the mixture of (3E,7E)-4,8,12-trimethyltrideca-3,7,11-trienoic acid and (3Z,7E)-4,8,12-trimethyltrideca-3,7,11-trienoic acid is subjected to a separation to obtain (3E,7E)-4,8,12-trimethyltrideca-3,7,11-trienoic acid;
c1) the (3E,7E)-4,8,12-trimethyltrideca-3,7,11-trienoic acid is subjected to a reduction to obtain (3E,7E)-homofarnesol (VI);
d1) the (3E,7E)-homofarnesol (VI) is subjected to a cyclization to obtain (-)-ambrox (VIII);
or
a2) by a process as defined in any one of claims 1 to 14, a mixture of (3E,7E)-4,8,12-trimethyltrideca-3,7,11-trienoic acid and (3Z,7E)-4,8,12-trimethyltrideca-3,7,11-trienoic acid is provided;
b2) the mixture of (3E,7E)-4,8,12-trimethyltrideca-3,7,11-trienoic acid and (3Z,7E)-4,8,12-trimethyltrideca-3,7,11-trienoic acid is subjected to a separation to obtain (3E,7E)-4,8,12-trimethyltrideca-3,7,11-trienoic acid;
c2) the (3E,7E)-4,8,12-trimethyltrideca-3,7,11-trienoic acid is subjected to a cyclization to obtain sclareolide (VII) ;
d2) the sclareolide (VII) is subjected to a reduction to obtain (-)-ambrox (VIII).

## Revendications

1. Procédé pour la préparation d'une composition, contenant au moins un acide carboxylique insaturé de formule générale (I) 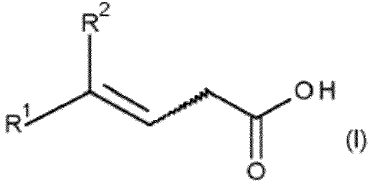 ou d'un sel correspondant, dans laquelle
R¹ représente hydrogène, C₁₋₂₄-alkyle linéaire ou ramifié, C₂₋₂₄-alcényle linéaire ou ramifié comportant 1, 2, 3 ou plus de 3 doubles liaisons C-C, C₅₋₁₂-cycloalkyle non substitué ou substitué par 1, 2 ou 3 radicaux C₁₋₆-alkyle ou aryle non substitué ou substitué par 1, 2 ou 3 radicaux C₁₋₆-alkyle,
R² représente hydrogène, C₁₋₂₄-alkyle linéaire ou ramifié, C₂₋₂₄-alcényle linéaire ou ramifié comportant 1, 2, 3 ou plus de 3 doubles liaisons C-C, ou
R¹ et R², conjointement avec l'atome de carbone auquel ils sont liés, représentent C₅₋₇-cycloalkyle non substitué ou C₅₋₇-cycloalkyle qui porte 1, 2 ou 3 radicaux C₁₋₆-alkyle linéaires ou ramifiés,
dans lequel on soumet un alcool allylique, choisi parmi des composés de formules générales (II.1) et (II.2) 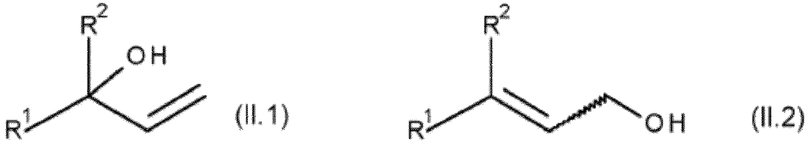 à une carbonylation par transformation avec du monoxyde de carbone en présence d'un catalyseur de type métal de transition, qui comprend au moins un métal des groupes 8, 9 ou 10 du système périodique des éléments, la transformation étant de plus réalisée en présence d'au moins un composé organique du phosphore en tant que ligand et en présence d'une quantité sous-stœchiométrique, par rapport à l'alcool allylique, d'un composé A) qui est choisi parmi des anhydrides d'acides monocarboxyliques aliphatiques en C₁₋₁₂, des anhydrides d'acides dicarboxyliques aliphatiques en C₄₋₂₀, des anhydrides d'acides dicarboxyliques cycloaliphatiques en C₇₋₂₀, des anhydrides d'acides dicarboxyliques aromatiques en C₈₋₂₀ et des alcools allyliques acylés des formules (III.1) et (III.2) 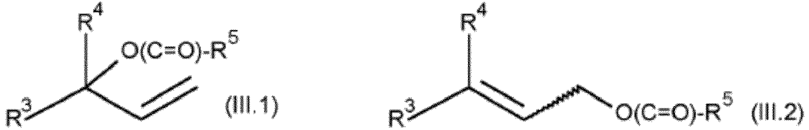 dans lesquelles
R³ représente hydrogène, C₁₋₂₄-alkyle linéaire ou ramifié, C₂₋₂₄-alcényle linéaire ou ramifié comportant 1, 2, 3 ou plus de 3 doubles liaisons C-C, C₅₋₁₂-cycloalkyle non substitué ou substitué par 1, 2 ou 3 radicaux C₁₋₆-alkyle ou aryle non substitué ou substitué par 1, 2 ou 3 radicaux C₁₋₆-alkyle,
R⁴ représente hydrogène, C₁₋₂₄-alkyle linéaire ou ramifié, C₂₋₂₄-alcényle linéaire ou ramifié comportant 1, 2, 3 ou plus de 3 doubles liaisons C-C, ou
R³ et R⁴, conjointement avec l'atome de carbone auquel ils sont liés, représentent C₅₋₇-cycloalkyle non substitué ou C₅₋₇-cycloalkyle qui porte 1, 2 ou 3 radicaux C₁₋₆-alkyle linéaires ou ramifiés,
R⁵ représente C₁₋₅-alkyle,
et la transformation étant réalisée à une température d'au plus 100 °C ;
ou
dans lequel on soumet un alcool allylique, choisi parmi des composés de formules générales (II.1) et (II.2) 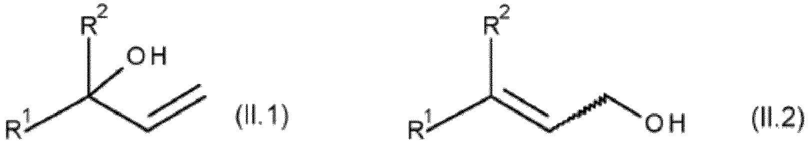 à une carbonylation par transformation avec du monoxyde de carbone en présence d'un catalyseur de type métal de transition, qui comprend au moins un métal des groupes 8, 9 ou 10 du système périodique des éléments, la transformation étant de plus réalisée en présence d'au moins un composé organique du phosphore en tant que ligand et en présence d'une quantité sous-stœchiométrique, par rapport à l'alcool allylique, d'un composé B) 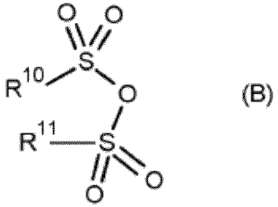 dans lequel
R¹⁰ et R¹¹, indépendamment l'un de l'autre, représentent C₁₋₆-alkyle, C₁₋₆-fluoroalkyle ou phényle, qui est non substitué ou substitué par un substituant choisi parmi brome, nitro et C₁₋₄-alkyle ;
et la transformation étant réalisée à une température d'au plus 100 °C.

2. Procédé selon la revendication 1, la transformation étant réalisée à une pression d'au plus 30 bars, de préférence d'au plus 25 bars, préférablement d'au plus 20 bars, en particulier d'au plus 15 bars.

3. Procédé selon l'une quelconque des revendications précédentes, la carbonylation n'étant pas réalisée en présence d'un halogénure d'hydrogène ajouté et pas en présence d'un halogénure de métal alcalin, d'un halogénure de métal alcalino-terreux ou d'un halogénure d'ammonium ajouté.

4. Procédé selon l'une quelconque des revendications précédentes, la teneur en halogénure du mélange réactionnel de la carbonylation n'étant pas supérieure à 2 % en moles, préférablement pas supérieure à 1 % en moles, par rapport à la teneur totale en alcool allylique des formules générales (II.1) et (II.2).

5. Procédé selon l'une quelconque des revendications précédentes, le composé A) étant choisi parmi l'anhydride acétique et l'acétate d'allyle, ou un ester de formule (III.1) ou (III.2) étant utilisé en tant que composé A), qui est issu de l'alcool de formule (II.1) ou (II.2) utilisé en tant qu'éduit pour la carbonylation.

6. Procédé selon l'une quelconque des revendications précédentes, dans lequel la transformation est réalisée en présence d'un réactif nucléophile choisi parmi une 4-(di(C₁₋₄-alkyl) amino) pyridine, une 4-(C_{1- 4}-alkyl)pyridine et une 4-(1-pyrrolidinyl)pyridine, préférablement la 4-(diméthylamino)pyridine.

7. Procédé selon l'une quelconque des revendications précédentes, dans lequel le métal de transition est utilisé pour la transformation en une quantité d'au plus 0,5 % en moles, préférablement d'au plus 0,3 % en moles, par rapport à la quantité totale en moles des composés (II.1) et (II.2).

8. Procédé selon l'une quelconque des revendications précédentes, le métal de transition étant choisi parmi Pd, Ru, Rh, Ir et Fe, préférablement Pd étant utilisé en tant que métal de transition.

9. Procédé selon l'une quelconque des revendications précédentes, le composé organique du phosphore étant choisi parmi des phosphines monodentates et bidentates, préférablement parmi des trialkylphosphines, des triarylphosphines, des dialkylarylphosphines, des alkyldiarylphosphines, des cycloalkyldiarylphosphines, des dicycloalkylarylphosphines, des tricycloalkylphosphines, des trihétérocyclylphosphines et des trihétarylphosphines, en particulier la triphénylphosphine, la di-tert-butylphénylphosphine, la cyclohexyldiphénylphosphine, la dicyclohexylphénylphosphine, la tri(p-tolyl)phosphine ou la tricyclohexylphosphine étant utilisée en tant que composé organique du phosphore.

10. Procédé selon l'une quelconque des revendications précédentes, dans lequel la quantité totale du composé A) est d'au plus 50 % en moles, préférablement d'au plus 30 % en moles, par rapport à la quantité totale en moles du composé (II.1) et (II.2).

11. Procédé selon l'une quelconque des revendications précédentes, la transformation étant réalisée à une température d'au plus 80 °C, préférablement d'au plus 75 °C.

12. Procédé selon l'une quelconque des revendications précédentes, dans lequel pour la transformation, un composé de formule générale (II.1) est utilisé, qui est choisi parmi le nérolidol, le linalool, le 3-méthyl-1-pentén-3-ol, le 1-heptén-3-ol et le 1-vinylcyclohexanol, en particulier l'E-nérolidol, ou dans lequel pour la transformation, le farnésol est utilisé en tant que composé de formule générale (II.2).

13. Procédé selon l'une quelconque des revendications précédentes, dans lequel la composition qui contient l'au moins un acide carboxylique insaturé de formule (I), est un mélange d'isomères E/Z de formule (I), contenant un acide 3-(E) de formule (I-E) et un acide 3-(Z) de formule (I-Z), 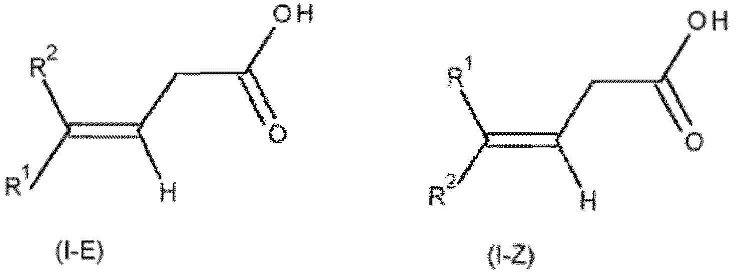
R¹ et R² présentant des significations différentes et R¹ présentant une priorité plus élevée selon l'UICPA ;
et on soumet ce mélange d'isomères E/Z de formule (I) à un enrichissement d'un isomère.

14. Procédé selon la revendication 13, dans lequel la composition qui contient l'au moins un acide carboxylique insaturé de formule (I), est un mélange d'isomères E/Z de formule (I), contenant un acide 3-(E) de formule (I-E) et un acide 3-(Z) de formule (I-Z),
dans lequel de plus
(1) la composition contenant le mélange d'isomères E/Z de formule (I) est soumise en présence d'un alcool et d'une enzyme de type lipase à une estérification catalysée par une enzyme, l'acide 3-(E) de formule (I-E) étant transformé au moins partiellement en un ester 3-(E), de sorte qu'une composition est obtenue, qui contient l'ester 3-(E), de l'acide 3-(E) non transformé de formule (I-E) et de l'acide 3-(Z) non transformé de formule (I-Z);
(2) la composition obtenue en (1) étant séparée, avec obtention d'une composition qui est appauvrie en acide 3-(E) de formule (I-E) et enrichie en acide 3-(Z) de formule (I-Z), et avec obtention d'une composition qui contient l'ester 3-(E) ;
(3) la composition obtenue en (2), qui est appauvrie en acide 3-(E) de formule (I-E) et enrichie en acide 3-(Z) de formule (I-Z), est soumise à une isomérisation pour l'augmentation de la teneur en acide 3-(E) de formule (I-E) ; et
(4) éventuellement l'ester 3-(E) obtenu en (2) étant clivé avec obtention de l'acide 3-(E) de formule (I-E) ;
ou dans lequel la composition qui contient l'au moins un acide carboxylique insaturé de formule (I), est un mélange d'isomères E/Z de formule (I), contenant un acide 3-(E) de formule (I-E) et un acide 3-(Z) de formule (I-Z), dans lequel de plus
(i) la composition contenant le mélange d'isomères E/Z de formule (I) est soumise à une estérification en présence d'un alcool avec obtention de l'ester 3-(E) et de l'ester 3-(Z) ;
(ii) l'ester 3-(E) et l'ester 3-(Z) obtenus en (i) sont soumis à une saponification enzymatique catalysée par une lipase, la lipase clivant l'ester 3-(E) au moins partiellement en l'acide 3-(E) de formule (I-E), une composition étant obtenue, qui contient l'acide 3-(E) du composé de formule (I-E), de l'ester 3-(E) non transformé et de l'ester 3-(Z) non transformé ;
(iii) la composition obtenue en (ii) est séparée avec obtention d'une composition qui contient l'acide 3-(E) du composé de formule (I-E) et avec obtention d'une composition, qui contient de l'ester 3-(E) non transformé et de l'ester 3-(Z) non transformé ;
(iv) la composition obtenue en (iii), qui contient de l'ester 3-(E) non transformé et de l'ester 3-(Z) non transformé, est soumise à un clivage d'ester, une composition étant obtenue, qui est appauvrie en acide 3-(E) de formule (I-E) ou en un sel de celui-ci et enrichie ou en acide 3-(Z) de formule (I-Z) ou en un sel de celui-ci ; et
(v) la composition obtenue en (iv) qui est appauvrie en acide 3-(E) de formule (I-E) et enrichie en acide 3-(Z) de formule (I-Z), est soumise à une isomérisation pour l'augmentation de la teneur en acide 3-(E) de formule (I-E).

15. Procédé pour la préparation de (-)-ambrox (VIII) 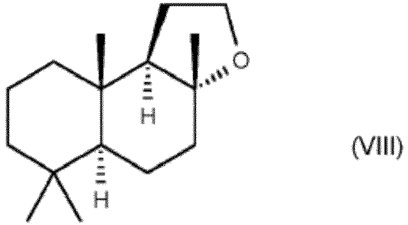 dans lequel
a1) selon un procédé tel que défini dans l'une quelconque des revendications 1 à 14, on met à disposition un mélange d'acide (3E,7E)-4,8,12-triméthyltridéca-3,7,11-triénique et d'acide (3Z,7E)-4,8,12-triméthyltridéca-3,7,11-triénique ;
b1) on soumet le mélange d'acide (3E,7E)-4,8,12-triméthyltridéca-3,7,11-triénique et d'acide (3Z,7E)-4,8,12-triméthyltridéca-3,7,11-triénique à une séparation avec obtention d'acide (3E,7E)-4,8,12-triméthyltridéca-3,7,11-triénique ;
c1) on soumet l'acide (3E,7E)-4,8,12-triméthyltridéca-3,7,11-triénique à une réduction avec obtention de (3E,7E)-homofarnésol (VI) ;
d1) on soumet le (3E,7E)-homofarnésol (VI) à une cyclisation avec obtention de (-)-ambrox (VIII) ;
ou
a2) selon un procédé tel que défini dans l'une quelconque des revendications 1 à 14, on met à disposition un mélange d'acide (3E,7E)-4,8,12-triméthyltridéca-3,7,11-triénique et d'acide (3Z,7E)-4,8,12-triméthyltridéca-3,7,11-triénique ;
b2) on soumet le mélange d'acide (3E,7E)-4,8,12-triméthyltridéca-3,7,11-triénique et d'acide (3Z,7E)-4,8,12-triméthyltridéca-3,7,11-triénique à une séparation avec obtention d'acide (3E,7E)-4,8,12-triméthyltridéca-3,7,11-triénique ;
c2) on soumet l'acide (3E,7E)-4,8,12-triméthyltridéca-3,7,11-triénique à une cyclisation avec obtention de sclaréolide (VII) ;
d2) on soumet le sclaréolide (VII) à une réduction avec obtention de (-)-ambrox (VIII).
